# FASCICULE DE BREVET EUROPEEN

(11) **EP 3 331 494 B1**
(45) Date de publication et mention de la délivrance du brevet: **21.04.2021**
(21) Numéro de dépôt: 16747777.7
(22) Date de dépôt: 04.08.2016
(51) Int. Cl.: A61K 9/00, A61K 47/02, A61K 47/14, A61K 9/107, A61K 47/44, A61K 31/704, A61K 49/04, A61K 49/08, A61K 49/10, A61P 35/00

(54) **COMPOSITION DESTINÉE À VECTORISER UN AGENT ANTICANCÉREUX**
ZUSAMMENSETZUNG ZUR VEKTORISIERUNG EINES KREBSMITTELS
COMPOSITION INTENDED TO VECTORISE AN ANTI-CANCER AGENT

(30) Priorité: 04.08.2015 FR 1557524
(43) Date de publication de la demande: 13.06.2018
(73) Titulaire: Guerbet, 93420 Villepinte (FR)
(72) Inventeur: ROBIC, Caroline, 94130 Nogent sur Marne (FR); MAYER, Jean-François, 93600 Aulnay sous Bois (FR)
(74) Mandataire: Lavoix
(86) Numéro de dépôt international: PCT/EP2016/068687
(87) Numéro de publication internationale: WO 2017/021508

(56) Documents cités:
- EP-A1- 2 077 106
- JP-A- 2005 068 058
- DATABASE MEDLINE [Online] US NATIONAL LIBRARY OF MEDICINE (NLM), BETHESDA, MD, US; septembre 1993 (1993-09), SHIONO T ET AL: "Efficacy of emulsion containing Gd-DTPA and lipiodol in hepatic transcatheter arterial embolization.", XP002756725, Database accession no. NLM8290693 & SHIONO ET AL.: "Efficacy of emulsion containing Gd-DTPA and lipiodol in hepatic transcatheter arterial embolization", RADIATION MEDICINE 1993 SEP-OCT, vol. 11, no. 5, septembre 1993 (1993-09), pages 187-190, ISSN: 0288-2043
- YI S W ET AL: "Stable lipiodolized emulsions for hepatoma targeting and treatment by transcatheter arterial chemoembolization", JOURNAL OF CONTROLLED RELEASE, ELSEVIER, AMSTERDAM, NL, vol. 50, no. 1-3, 2 janvier 1998 (1998-01-02), pages 135-143, XP004107645, ISSN: 0168-3659, DOI: 10.1016/S0168-3659(97)00127-2

## Description

La présente invention concerne une composition sous forme d'émulsion eau-dans-huile comprenant un agent anticancéreux, un agent densifiant choisi parmi les complexes de chélate macrocyclique non-ionique avec un métal paramagnétique, une huile iodée et un tensioactif.

Depuis plus d'un siècle, les huiles iodées comme le produit Lipiodol® sont utilisées en tant que produit de contraste dans des examens radiologiques tels que la lymphographie et pour le diagnostic de lésions hépatiques. Le Lipiodol® est constitué principalement d'esters éthyliques d'acides gras iodés de l'huile d'œillette.

Depuis près de trente ans, ces huiles iodées sont utilisées dans des procédures de radiologie interventionnelle. Le Lipiodol® se caractérise par sa propension à être capté sélectivement par les tumeurs hépatiques. Il a donc été proposé comme vecteur d'agent anticancéreux pour le traitement du carcinome hépatocellulaire dans une technique qui s'appelle la chimioembolisation intra-artérielle ou « TransArterial ChemoEmbolisation » (TACE) en anglais (Nakamura et al.: Radiology, 1989 ; 170:783-6 et J.M. Idée - B. Guiu : Critical Reviews in Oncology/Hematology, 2013 ; 88(3):530-49). Les huiles iodées et en particulier le Lipiodol® sont aussi connues pour induire une embolisation transitoire de la circulation artérielle provoquant ainsi un ralentissement de celle-ci. Etant donné que la plupart des agents anticancéreux sont solubles dans l'eau, la forme « émulsion », qui est adaptée pour le mélange de deux phases non solubles l'une dans l'autre, apparait comme étant la plus judicieuse pour mélanger une huile iodée et un agent anticancéreux. Elle semble être la plus adaptée pour transporter et délivrer dans une tumeur un agent anticancéreux, trop toxique et pas assez efficace quand il est administré non émulsifié par voie intra-artérielle ou par voie systémique.

JP 2005/068058 décrit un agent de contraste sous forme d'émulsion huile-dans-eau ou eau-dans-huile-dans-eau, comprenant un agent de contraste pour rayons X et un métal paramagnétique utile pour des analyses de tomodensitométrie ou d'imagerie par résonance magnétique.

Shiono et al. (Radiation Medicine, 1993, 187-190) décrivent l'évaluation clinique d'une émulsion comprenant du Lipiodol®, du Gd-DTPA et des anticancéreux. Cette émulsion peut être suivie par tomodensitométrie ou imagerie par résonance magnétique.

EP 2 077 106 décrit des émulsions eau-dans-huile-dans-eau dans lesquelles la phase aqueuse interne contient une substance ionique physiologiquement active et un composé physiologiquement acceptable ayant un poids moléculaire de 1 000 ou moins et générant un contre-ion polyvalent à deux valences ou plus pour la substance ionique physiologiquement active. Ces émulsions permettent d'encapsuler des substances actives dans la phase aqueuse interne.

Yi et al. (Journal of Controlled Release, 1998, 50, 135-143) décrit des émulsion eau-dans-huile comprenant du Lipiodol®, de la doxorubicine et un dérivé poly(oxyde d'éthylène) d'huile de ricin hydrogénée.

Une émulsion « eau-dans-huile » émulsion dite « inverse » est une émulsion que l'on note E/H en français ou W/O (water in oil) en anglais. Il s'agit d'une dispersion de gouttelettes de phase aqueuse dans une phase lipidique. Une émulsion « huile dans eau» est une émulsion dite « directe» que l'on note H/E en français ou O/W (oil in water) en anglais. A l'opposé des émulsions E/H, il s'agit alors d'une dispersion de gouttelettes de phase lipidique dans une phase aqueuse. On parle de « sens d'émulsion » quand on se réfère à la nature E/H ou H/E d'une émulsion.

Les émulsions huile-dans-eau (H/E, ou O/W en anglais), qui comprennent l'agent anticancéreux dans la phase continue aqueuse, présentent l'inconvénient important de libérer rapidement l'agent anticancéreux dans le sang. Une partie non négligeable de l'agent thérapeutique n'atteint donc pas le site ciblé, ce qui peut, d'une part, induire une toxicité systémique et d'autre part réduire l'efficacité de cet agent thérapeutique. De plus, ce type d'émulsion H/E présente le risque de provoquer une embolie pulmonaire voire cérébrale. Ce risque est augmenté quand la taille des gouttelettes d'huile de ces émulsions est inférieure à 10 µm. Ce deuxième inconvénient est difficile à écarter puisqu'en augmentant la taille des gouttelettes, l'instabilité de ces émulsions est augmentée.

Les émulsions eau-dans-huile (E/H, ou W/O en anglais), aussi appelées « émulsions inverses », et comprenant une huile iodée et un agent anticancéreux, sont moins évoquées dans la littérature que les émulsions H/E. Elles sont décrites comme libérant plus lentement l'agent thérapeutique dans la tumeur et comme présentant une viscosité plus importante que les émulsions huile-dans-eau (De Baere et al., Radiology 1995 ; 194:165-170). Ces raisons incitent à choisir une forme d'émulsion E/H pour vectoriser un agent anticancéreux au sein d'une tumeur. Toutefois, ces émulsions E/H n'ont pas toujours une efficacité suffisante du fait de leur manque de stabilité au contact du sang et des bifurcations vasculaires en amont de la tumeur. En effet, pour augmenter le ciblage tumoral des agents anticancéreux et améliorer en même temps l'efficacité thérapeutique et la tolérance du traitement, une émulsion doit rester stable jusqu'au moment où elle atteint la tumeur et sa répartition dans la lésion tumorale doit être complète et homogène.

Différentes solutions de stabilisation d'émulsions ont ainsi été proposées dans l'art antérieur. Pour stabiliser des émulsions H/E, de nombreux auteurs ont proposé l'utilisation de tensioactif à HLB élevée (plus de 8).

L'utilisation de tensio-actif à HLB élevée voire très élevée comme les esters d'acides gras et de sorbitane polyoxyéthylénés, monostéarate de sorbitane polyoxyéthylénés ou polysorbate 60 (Montanox® 60, HLB = 14,9) et monolaurate de sorbitane polyoxyéthyléné ou polysorbate 20 (Montanox 20®, HLB = 16,7) a été décrite pour la préparation d'émulsions huile-dans-eau à base d'idarubicine et de Lipiodol® stables 6 mois.

JPH0647559 décrit une émulsion H/E comprenant entre 10 et 30% de Lipiodol®, un agent anticancéreux et entre 0,1 et 2% d'un tensioactif hydrophile, le HCO-60 autrement appelé huile de ricin hydrogénée polyoxyéthylenée (Polyoxyéthylene hydrogenated castor oil en anglais) (HLB = 14). Il s'agit *a priori* d'un PEG-60 lié à un acide ricinoléique.

Les demandes EP 0 294 534 et EP 0 581 842 font référence à d'autres documents. On apprend notamment que DE 26 02 907 décrit une émulsion huile-dans-eau contenant entre 50 et 60% de triglycérides iodés, entre 2 et 10% d'esters d'acides gras de polyoxyéthylène sorbitan (HLB = 13 à 17) et entre 2 et 40% d'eau. Grimes et al. (J. Pharm. Sci. 1979 Jan;68(1):52-6) décrit l'utilisation de polysorbate 80 (HLB = 15), de sorbitan mono-oléate (HLB = 8,6) et de phosphatidylcholine pour obtenir des émulsions comprenant de l'huile iodée. Vermess et al. a décrit des émulsions (US 4,404,182 ou J. Comput. Assist. Tomogr. 3 : 25-31, 1979) contenant 53% (v/v) de Lipiodol®, 10% d'alcool et 0,45% de lécithine de soja. Ces émulsions huile-dans-eau ont des tailles de particules de 2 à 3 µm. Schumacher et al. (Europ. J. Radiol. 5, 167-174, 1985) décrit différentes émulsions contenant des huiles iodées et préparées à l'aide d'émulsifiants comme le polyoxyethylène-4-sorbitan monolaurate (Tween® 80, Serva : HLB = 15,3), glycérol Polyéthylene glycol ricinoléate (Crémophor® EL : HLB = 14,5), diacetylphosphate DP (Sigma), lécithine issu d'œufs (Fluka GmbH), Doxypolygélatine (Gelinfundol® 5,5% Biotest GmbH) et dextran 60 (Macrodex® 4,5%, RL Knoll).

L'utilisation d'amiodarone (médicament antiarythmique de formule chimique 2-butyl-3 - benzofuranyl)[4-[2-(diéthylamino)éthoxy]-3,5-diiodophényl]méthanone) a permis de stabiliser une émulsion huile-dans-eau de Lipiodol® (44% (v/v)) et de doxorubicine ou de pirarubicine, jusqu'à quatre semaines à 37°C. Cette propriété est due à la présence d'un excipient dans ce médicament, le polysorbate 80, un émulsifiant à HLB haute (Boulin et al., Digestive and Liver Disease 43 (2011) 905 - 911). Des travaux complémentaires de la même équipe ont permis de montrer que l'amiodarone n'améliore quasiment pas la stabilité d'une émulsion à base de Lipiodol® et d'idarubicine et ne semblait pas majorer la cytotoxicité de l'agent anticancéreux. L'utilisation seule d'idarubicine et de Lipiodol® est donc même conseillée.

Dans Nakamura et al. (Radiology, 1989 ; 170:783-6) est montrée l'apparence visuelle de différentes émulsions obtenues en mélangeant 1 mL d'eau distillée comprenant un produit de contraste ionique, le diatrizoate de sodium et de méglumine (Hypaque®, Gastrografin® ou Urografin®) et 3 mL de Lipiodol® (figure 1). Il est indiqué que l'émulsion C n'a pas déphasé après 24h mais il peut être aisément constaté sur cette figure que cette émulsion n'est en réalité pas stable, la partie inférieure du tube qui la contient, étant plus limpide que la partie supérieure de celui-ci. Dans ce document est aussi décrite la préparation d'émulsion de Lipiodol® et de doxorubicine ou de mitomycine dans des ratio 2-3/1. Il est indiqué que l'émulsion obtenue est une émulsion E/H. Il est souligné la moindre libération de l'agent anticancéreux dans le cas de l'utilisation de cette émulsion (figure 2). Le pic plasmatique visualisé après injection de cette émulsion reste toutefois non négligeable. Cette émulsion ne doit pas être suffisamment stable puisqu'il n'y a pas utilisation de tensioactif. En effet, 2 minutes après injection intra-artérielle de leur émulsion, est observée une concentration plasmatique de la doxorubicine plus faible de 83% (((3500-600)/3500) x 100) par rapport à la concentration plasmatique mesurée après injection de cet agent anticancéreux seul. A 5 minutes, cette diminution est de 80%.

Dans Raoul et al. (Cancer, 1992, vol. 70, n°3, 585-90), sont décrites des émulsions comprenant 50 mg de doxorubicine faites en mélangeant 10 mL de Lipiodol® et 2,5 mL d'ioxaglate (Hexabrix®). Les émulsions obtenues, dont le sens E/H ou H/E n'est pas précisé, provoquent un pic plasmatique significativement inférieur à celui provoqué par l'injection intra-artérielle de doxorubicine seule. Toutefois, ce pic plasmatique indique un passage non négligeable de l'agent anticancéreux dans le sang. En effet, 2 minutes après injection intra-artérielle de ces émulsions, est observée une concentration plasmatique de la doxorubicine plus faible de 59% (((2200 - 900)/2200) x 100) par rapport à la concentration plasmatique mesurée après injection de cet agent anticancéreux seul. Le calcul correspondant à 5 minutes après injection est encore plus défavorable puisque cette diminution n'est alors que de 33% (((1050 - 700)/1050) x 100). Quand une embolisation est pratiquée après injection de ces émulsions, ces diminutions sont à 2 et 5 minutes respectivement de 82% (((2200 - 400)/2200) x 100) et de 43% (((700-400)/700) x 100).

Ces différentes émulsions, quand elles sont sous forme « huile-dans-eau » ont une capacité de vectorisation des agents anticancéreux insuffisante et cela, même si elles ont été stabilisées à l'aide de tensioactif à HLB élevée et leur utilisation présente toujours un risque important d'embolie. Cette capacité de vectorisation insuffisante s'explique par la nature même de l'émulsion puisque dans le cas des émulsions huile-dans-eau, l'agent anticancéreux, le plus souvent hydrosoluble, se trouve dans la phase continue aqueuse et est donc très rapidement dilué dans la circulation sanguine. En outre, plusieurs de ces émulsions contiennent des émulsifiants synthétiques tels que des Tween® (HLB élevée) ou des Span® (HLB soit faible, soit élevée), émulsifiants listés dans la pharmacopée européenne, qui engendrent des effets secondaires. Les polysorbates comme les Tween® sont décrits comme potentiellement toxiques. Les esters de sorbitane tels que les Span® ne sont pas préconisés pour une utilisation en injection parentérale. (Handbook of Pharmaceutical Excipients, 2009).

Fréquemment, des émulsions décrites dans des publications comme des émulsions « eau-dans-huile » ne sont pas des émulsions de cette nature. C'est le cas par exemple des émulsions décrites dans la publication de Yi et al. (Journal of Controlled Release 50 (1998) 135-143) ou de l'émulsion intermédiaire décrite dans Higashi et al. (Advances Drug delivery Reviews 45 (2000), 57-64). Quand elles sont réellement sous la forme E/H, ces émulsions ont des stabilités et des capacités de vectorisation d'agent anti-cancéreux insuffisantes. Elles ont donc une efficacité insuffisante après injection puisqu'une part importante de la quantité d'agent anticancéreux injectée par voie intra-artérielle n'arrive pas au niveau de la lésion ciblée (Raoul et al., 1992).

Pour préparer la majorité des émulsions décrites dans l'art antérieur, sont utilisés des produits de contraste iodés, comme agent densifiant, préférentiellement des produits de contraste iodés non-ioniques. Ces produits de contraste, qui sont présents dans la même phase que l'agent anticancéreux (i.e. la phase aqueuse), sont visualisables par des techniques d'imagerie par rayons X. Or l'huile iodée présente dans la phase huileuse est également visualisable par cette modalité d'imagerie. Le fait d'avoir une augmentation du contraste aussi bien dans la phase aqueuse que dans la phase huileuse empêche la vérification par une technique d'imagerie de la colocalisation de l'agent anticancéreux et de l'agent densifiant.

La Demanderesse a mis au point une composition permettant de réaliser une émulsion eau-dans-huile comprenant un agent anti-cancéreux stable pendant au moins 24h à 20°C et présentant généralement une capacité de vectorisation améliorée par rapport aux émulsions de l'art antérieur tout en permettant un double contrôle plus précis de son efficacité par une technique d'imagerie par résonance magnétique (IRM) et par une technique d'imagerie par rayons X.

Cette émulsion a donc trois grands avantages : elle est aisément utilisable dans un contexte hospitalier, sa stabilité lui permettant d'être préparée au moins 24 heures à l'avance dans l'officine de l'hôpital, elle présente un risque très limité pour le patient, tout en ayant une efficacité thérapeutique améliorée et son efficacité est plus facilement mesurable par une technique d'imagerie que l'efficacité des émulsions classiques de l'art antérieur.

Cette émulsion a aussi comme avantage de permettre de corréler une quantité d'huile iodée (e.g. Lipiodol®) présente dans une tumeur, qui pourra être estimée par une méthode d'imagerie par rayons X, à une quantité d'agent anticancéreux présente effectivement dans la tumeur. Pour la majorité des émulsions de l'art antérieur, la quantité d'huile iodée que l'on estime présente dans une tumeur n'est en aucun cas indicatrice de la quantité d'agent anticancéreux présente dans cette tumeur. L'émulsion selon l'invention permet donc de diminuer les faux-positifs lorsque l'on cherche à vérifier que l'agent anticancéreux a effectivement été administré au cœur de la tumeur et elle permet aussi de mieux vérifier l'efficacité de celle-ci par une autre modalité d'imagerie, telle que l'imagerie par résonance magnétique.

Ainsi l'invention a comme objet une composition sous forme d'émulsion eau-dans-huile comprenant :
- de 20 à 40% (v/v) de phase aqueuse, préférentiellement de 20 à 35% (v/v), plus préférentiellement 25% (v/v) de phase aqueuse, sous forme de gouttelettes, comprenant un agent anticancéreux et un agent densifiant choisi parmi les complexes de chélate macrocyclique non-ionique avec un métal paramagnétique, préférentiellement avec un lanthanide, plus préférentiellement avec du gadolinium,
- de 60 à 80%, préférentiellement de 65 à 80% (v/v), plus préférentiellement 75% (v/v) de phase lipidique comprenant une huile iodée et au moins un tensioactif de formule (I) dans une proportion, en masse de tensioactif par rapport au volume total de la composition, de 0,3 à 5%, préférentiellement de 0,5 à 2%, plus préférentiellement de 1%, la formule (I) dudit tensioactif étant la suivante: 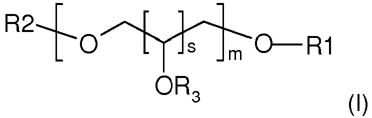 dans laquelle :
   - s vaut 0 ou 1,
   - m représente un nombre entier de 2 à 30,
   - R₁ représente un groupement de formule (II) 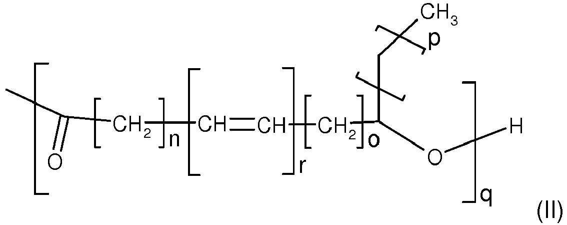
   dans laquelle n représente un nombre entier de 4 à 10, o représente un nombre entier de 1 à 4, p représente un nombre entier de 3 à 7, q représente un nombre entier de 2 à 10 et r vaut 0 ou 1,
   - R₂ représente un atome d'hydrogène ou est identique à R₁, et
   - chaque R₃ représente indépendamment un atome d'hydrogène ou est identique à R₁.

De préférence, dans la formule (I) ci-dessus, chaque R₃ représente un atome d'hydrogène. La formule (I) dudit tensioactif a alors la formule (I') suivante:

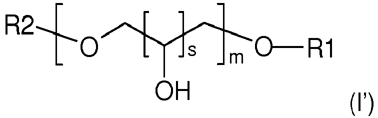

La proportion de tensioactif est exprimée en masse de tensioactif sur le volume total de la composition sous forme d'émulsion. Les proportions en phases aqueuse ou lipidique sont exprimées en volume de la phase sur le volume total de la composition sous forme d'émulsion.

Cette composition est destinée à vectoriser un agent anticancéreux. L'invention concerne également l'utilisation de cette composition comme vecteur d'un agent anticancéreux.

Cette composition est sous forme d'une émulsion eau-dans-l'huile (aussi appelée « émulsion inverse » ou E/H ou W/O). Une telle émulsion est constituée d'une phase lipidique et d'une phase aqueuse dispersée sous forme de gouttelettes. L'huile iodée de la composition se situe dans la phase lipidique. Le tensioactif de formule (I) ou (I') se situe à l'interface entre les phases aqueuse et lipidique. Au sens de la présente demande, pour le calcul des proportions de phases aqueuse et lipidique, on considèrera que le tensioactif se situe dans la phase lipidique.

En particulier, les modes de réalisations suivants sont avantageux:

| | % (v/v) de phase aqueuse sous forme de gouttelettes comprenant un agent anticancéreux | % (v/v) de phase lipidique comprenant une huile iodée | % (m/v) d'au moins un tensioactif |
|---|---|---|---|
| Composition sous forme d'émulsion selon l'invention | 20 - 35 | 65 - 80 | 0,5 - 2 |
| Composition sous forme d'émulsion selon l'invention | 25 | 75 | 1 |

L'émulsion selon l'invention est avantageusement stable. Par émulsion « stable », on entend une émulsion présentant, dans des conditions classiques de température (20°C) et de pression atmosphérique (1 bar) et dans les 24 heures suivant sa préparation, un déphasage visuel de moins de 5% en volume par rapport à la totalité de la composition sous forme d'émulsion. Préférentiellement, par émulsion « stable », on entend une émulsion ne présentant aucun déphasage visuel dans les conditions évoquées ci-dessus et dans les 24 heures suivant sa préparation. Un déphasage visuel se manifeste au moment où une solution n'apparait plus homogène à l'œil, c'est-à-dire au moment où l'on voit l'apparition d'au moins deux phases.

De manière plus préférentielle, par émulsion « stable », on entend une émulsion dont la taille moyenne des gouttelettes varie de moins de 10%, notamment de moins de 5%, de préférence dont la taille moyenne des gouttelettes ne varie pas, où la taille moyenne est mesurée au microscope optique (par exemple le microscope LEICA DM2000 LED) 24 heures après sa préparation.

De préférence, l'injection intra-artérielle de l'émulsion selon l'invention induit une diminution de la concentration plasmatique de l'agent anticancéreux entre 0 et 5 minutes suivant cette injection de plus de 90%, préférentiellement de plus de 94%, plus préférentiellement de plus de 97%, encore plus préférentiellement de plus de 99% par rapport à l'injection intra-artérielle de l'agent anticancéreux seul. De manière avantageuse, ces concentrations plasmatiques et cette diminution sont confirmées par des mesures de cinétiques plasmatiques selon des protocoles connus de l'homme du métier.

L'expression de la différence entre un pic de concentration plasmatique d'un agent anticancéreux après injection d'un produit particulier comprenant cet agent et celui obtenu après injection de l'agent anticancéreux seul est notamment évoquée par Hong et al. (Clin. Cancer Res. 2006 : 12(8)).

Lorsque l'émulsion comprend moins de 20% (v/v) de phase aqueuse, l'agent anticancéreux est difficilement solubilisable dans celle-ci. Lorsque l'émulsion comprend plus de 40% de phase aqueuse, la viscosité de la composition sous forme d'émulsion est trop forte. En effet, en augmentant la concentration en gouttelettes de phase aqueuse dans la phase continue lipidique comprenant une huile iodée, on augmente la viscosité de la composition globale.

La phase aqueuse comprend un agent anticancéreux à une dose thérapeutiquement efficace. Par « dose thérapeutiquement efficace », on entend une dose permettant de traiter un cancer ou d'en freiner l'évolution. Préférentiellement, dans le cas où l'agent anticancéreux est choisi parmi les anthracyclines, une dose thérapeutiquement efficace représente une quantité d'agent anticancéreux de 20 à 150 mg, plus préférentiellement de 50 à 100 mg.

La densité de la phase lipidique est préférentiellement de 1,15 à 1,30, plus préférentiellement de 1,20 à 1,30, encore plus préférentiellement de 1,28. De manière préférentielle, la phase aqueuse et la phase lipidique ont la même densité (en d'autres termes, elles sont de densité égale) ou des densités jusqu'à 5% différentes l'une de l'autre.

L'agent densifiant permet de réaliser une densification de la phase aqueuse comprenant un agent anticancéreux. Pour diminuer la densité de la phase lipidique comprenant une huile iodée, une seconde huile de densité inférieure à 1 peut être ajoutée (on réalise alors une « dé-densification » de la phase lipidique comprenant une huile iodée).

### Complexes de chélate macrocyclique non-ionique avec un métal paramagnétique

Dans un mode de réalisation avantageux, les complexes de chélate macrocyclique non-ionique avec un métal paramagnétique sont choisis parmi le Gd-HP-DO3A (Dénomination Commune Internationale : Gadoteridol, nom commercial : ProHance®), le Gd-BT-DO3A (DCI : Gadobutrol, nom commercial : Gadovist®), un complexe de formule (XI) : 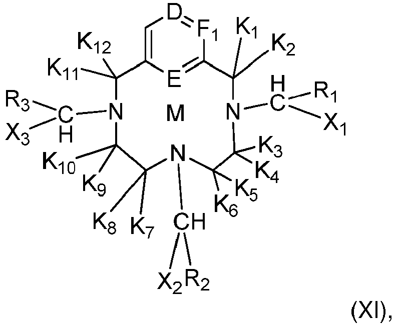 dans laquelle R₁, R₂ et R₃ représentent -COOH,
X₁, X₂ et X₃ représentent indépendamment l'un de l'autre L-Y dans laquelle L représente un groupe alkylène en C₁-C₃, de préférence (CH₂)ₙ avec n = 1 à 3, Y représente -CONH₂, -CO-NR₇R₈ ou -NR₇-CO-R₈, dans lesquelles R₇ représente H ou un groupe alkyle en C₁-C₆ ou un groupe hydroxyalkyle en C₁-C₈, notamment en C₂-C₆, par exemple en C₂-C₄, avantageusement -CH₂-CH₂OH, -CHOH-CH₂OH, -CH-(CH₂OH)₂, -(CH₂)ₘ-(CHOH)ₚ-CH₂OH avec m = 1 à 3 et p = 1 à 4 ou -C-(CH₂OH)₃ et R₈ représente un groupe alkyle en C₁-C₆ ou hydroxyalkyle en C₁-C₈, notamment en C₂-C₆, par exemple en C₂-C₄, avantageusement -CH₂-CH₂OH, -CHOH-CH₂OH, -CH-(CH₂OH)₂, - (CH₂)ₘ-(CHOH)ₚ-CH₂OH avec m = 1 à 3 et p = 1 à 4 ou -C-(CH₂OH)₃, à la condition qu'au moins R₇ ou R₈ représente un groupe hydroxyalkyle en C₁-C₈ ;
D représente CH ou N ;
E représente CH ou N ;
F₁ représente CH ou N ;
K₁ à K₁₂ représentent chacun indépendamment H, -(CH₂)ⱼ-CH₃ ou -(CH₂)ᵢ-OH dans lesquels j = 0 à 3 et i = 1 à 3, avantageusement H, ou K₃ ou K₄ avec K₅ ou K₆, et/ou K₇ ou K₈ avec K₉ ou K₁₀ forment avec les atomes de carbone auxquels ils sont liés un cycle ayant 3 à 6 atomes de carbone ; et
M représente un ion d'un métal paramagnétique ;
ou un énantiomère, ou un diastéréoisomère (préférentiellement choisi parmi les diastéréoisomères RRS, RSR, RSS) de ceux-ci ou leurs mélanges,
et un mélange de ceux-ci.

Lorsque R₇ et/ou R₈ représentent -(CH₂)ₘ-(CHOH)ₚ-CH₂OH avec m = 1 à 3, p = 1 à 4, chacune des combinaisons entre m = 1, 2, 3 et p = 1, 2, 3, 4, notamment m = 2 et p = 4, m = 3 et p = 4, ou m = 3 et p = 3 est possible.

De préférence, lorsque R₇ et/ou R₈ représentent un groupe hydroxyalkyle, ils représentent un hydroxyalkyle en C₁-C₆, avantageusement -CH₂-CH₂OH, -CHOH-CH₂OH, -CH-(CH₂OH)₂, -(CH₂)ₘ-(CHOH)ₚ-CH₂OH avec m = 1 à 3, p = 1 à 4 et m+p = 2 à 5 ou -C-(CH₂OH)₃

De manière préférentielle, les complexes de chélate macrocyclique non-ionique avec un métal paramagnétique ont une densité de 1,10 à 1,30, plus préférentiellement de 1,20 à 1,30, encore plus préférentiellement de 1,28.

Dans un mode de réalisation particulier, la densité du chélate macrocyclique non-ionique avec un métal paramagnétique est augmentée jusqu'à une valeur maximale limite de 1,30, soit par évaporation si ce composé est sous forme liquide, soit par concentration si ce composé est sous forme de poudre.

Ces complexes de chélate macrocyclique non-ionique avec un métal paramagnétique ont comme avantage de permettre une bonne solubilité de l'agent anticancéreux dans la phase aqueuse et de ne pas déstabiliser l'émulsion.

### Complexe de formule (XI)

On préfère particulièrement les complexes de formule (XI) pour lesquels les trois chaînes Y ont chacune un poids moléculaire inférieur à 200 g/mol, avantageusement entre 50 et 100 g/mol, et notamment les composés pour lesquels les chaînes Y comprennent chacune 1 à 5 groupes OH.

Selon des réalisations avantageuses, le complexe de formule (XI) est tel que E représente un atome N et D et F₁ représentent CH.

Selon des modes de réalisations avantageux, le complexe de formule (XI) est tel que X₁, X₂ et X₃ représentent indépendamment -(CH₂)n-CO-NR₇R₈ ou -(CH₂)n-NR₇-CO-R₈, dans lesquelles n est compris entre 1 et 3, R₇ représente H ou un groupe méthyle, R₈ représente un groupe hydroxyalkyle en C₁-C₆, avantageusement en C₂-C₃, de préférence -CH₂-CH₂OH, -CHOH-CH₂OH, -CH-(CH₂OH)₂, -CH₂-(CHOH)ₚ-CH₂OH avec p = 1 à 4 ou - C-(CH₂OH)₃. Avantageusement, X₁ à X₃ représentent indépendamment -(CH2)n-CONR₇R₈ dans lesquelles n est compris entre 1 et 3, R₇ représente H ou un groupe méthyle, R₈ représente un groupe hydroxyalkyle en C₁-C₄, de préférence -CH₂-CH₂OH, - CHOHCH₂OH, -CH-(CH₂OH)₂, -CH₂-(CHOH)ₚ-CH₂OH avec p =1 ou 2 ou -C-(CH₂OH)₃ De façon avantageuse X₁ à X₃ représentent indépendamment -(CH₂)N-CONR₇R₈, dans lesquelles n est compris entre 1 et 3, R₇ représente H, R₈ représente -CH₂-CH₂OH, - CHOH-CH₂OH, -CH-(CH₂OH)₂, -CH₂-(CHOH)p-CH₂OH avec p =1 à 4 ou -C-(CH₂OH)₃.

Préférentiellement, le complexe de formule (XI) est choisi parmi les complexes entre un ligand de formule (XI') et (XI") : 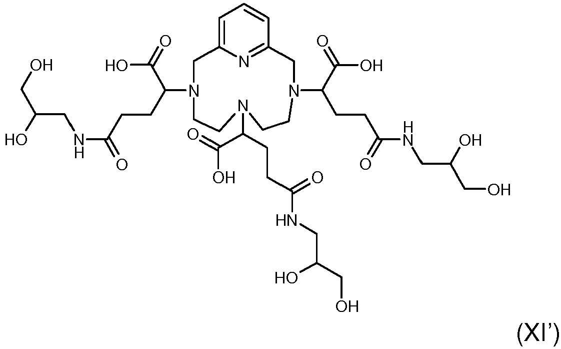 ou 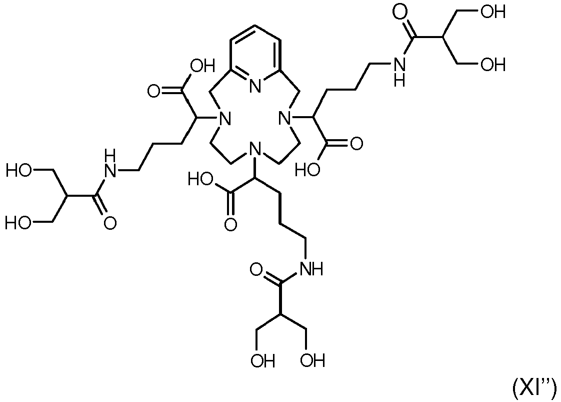 et un ion de métal paramagnétique M.

De manière avantageuse, l'ion de métal paramagnétique M est choisi parmi les ions d'un métal paramagnétique de nombre atomique 21-29, 42-44 ou 58-70, c'est-à-dire parmi les ions de scandium (Sc), titane (Ti), vanadium (V), chrome (Cr), manganèse (Mn), fer (Fe), cobalt (Co), nickel (Ni), cuivre (Cu) ou les ions de molybdène (Mo), technétium (Tc), ruthénium (Ru) ou les ions de cérium (Ce) praséodyme (Pr), néodyme (Nd), prométhium (Pm), samarium (Sm), europium (Eu), gadolinium (Gd), terbium (Tb), dysprosium (Dy), holmium (Ho), erbium (Er), thulium (Tm), ytterbium (Yb). L'ion de métal paramagnétique M est préférentiellement choisi parmi les ions de manganèse, de fer et de lanthanides, plus préférentiellement choisi parmi les ions Mn²⁺, Fe³⁺ et les ions de gadolinium comme Gd³⁺ et encore plus préférentiellement choisi parmi les ions de lanthanides et notamment les ions de gadolinium comme Gd³⁺.

De manière préférentielle, les complexes de chélate macrocyclique non-ionique avec un métal paramagnétique sont choisis parmi les complexes de chélate macrocyclique non-ionique avec un ion de gadolinium (tel que Gd³⁺). De manière plus préférentielle, les complexes de chélate macrocyclique non-ionique avec un ion de gadolinium sont choisis parmi le Gd-HP-DO3A, le Gd-BT-DO3A et un complexe de formule (XI) évoquée ci-dessus dans laquelle M est un ion de gadolinium. De manière encore plus préférentielle, les complexes de chélate macrocyclique non-ionique avec un ion de gadolinium sont choisis parmi le Gd-BT-DO3A et un complexe de formule (XI) évoquée ci-dessus dans laquelle M est un ion de gadolinium.

De manière préférentielle, le complexe de formule (XI) évoquée ci-dessus dans laquelle M est un ion de gadolinium est choisi parmi les complexes de formule: 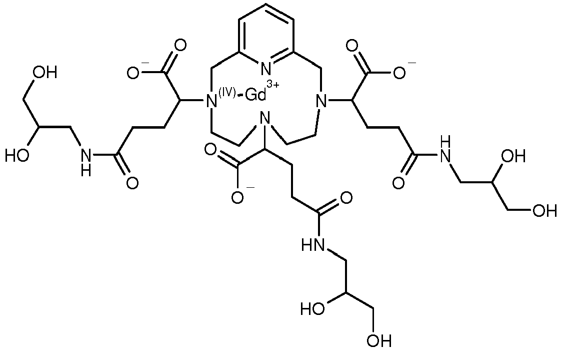 ou

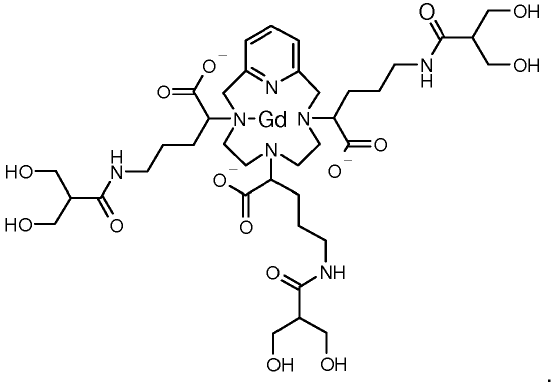

Les procédés de synthèse de ces complexes de formule (XI) sont bien connus de l'homme du métier, et sont notamment décrits dans le document EP 1 931 673. Ces complexes de formule (XI) peuvent être formulés avec par exemple un complexe calcique de DOTA comme décrit dans le document WO 2014/174120.

Dans un mode de réalisation avantageux, la phase aqueuse peut comprendre en outre comme agent densifiant, un produit de contraste iodé non-ionique jusqu'à 70 % en volume par rapport au volume total d'agent densifiant présente dans la composition. Le produit de contraste iodé non ionique, utilisable en tant qu'agent densifiant en combinaison avec le complexe de chélate macrocyclique non-ionique avec un métal paramagnétique est, de manière préférée, choisi parmi l'iobitridol (Xenetix®), l'iopamidol (Iopamiron®, Isovue®), l'iomeprol (Iomeron®), l'ioversol (Optiray®, Optiject®), l'iohexol (Omnipaque®), l'iopentol (Imagopaque®), l'ioxitol (Oxilan®), l'iopromide (Ultravist®), le metrizamide (Amipaque®), l'iosarcol (Melitrast®), l'iotrolan (Isovist®), l'iodixanol (Visipaque®), l'iosiménol et l'iosimide (Univist®) et un mélange de ceux-ci. L'iobitridol est le produit iodé non ionique préférentiel. Les produits Xenetix® 250, Xenetix® 300, Xenetix® 350 ont respectivement des densités de 1,28, 1,34 et 1,40. Ces produits de contraste iodés non-ioniques ont comme avantage de permettre une bonne solubilité de l'agent anticancéreux dans la phase aqueuse et de ne pas déstabiliser l'émulsion. De manière préférentielle, la composition comprend comme agent densifiant entre 30 et 50% en volume d'un complexe de chélate macrocyclique non-ionique avec un métal paramagnétique et entre 50 à 70% en volume d'un produit de contraste iodé non-ionique, ces pourcentages étant précisés par rapport au volume total d'agent densifiant dans la composition.

L'utilisation de produits de contraste iodés ioniques comme l'acide ioxaglique (Hexabrix®) ou le diatrizoate de méglumine et/ou de sodium (Hypaque®, Gastrografin®, Gastroview® ou Urografin®) n'est pas indiquée puisque ces produits de contraste ont comme inconvénient de réduire la solubilité de l'agent anticancéreux dans la phase aqueuse, voire d'empêcher leur solubilisation et/ou d'augmenter l'osmolalité des compositions.

Dans un autre mode de réalisation avantageux (qui peut être combiné ou non avec le mode de réalisation ci-dessus dans lequel la phase aqueuse comprend comme agent densifiant une certaine proportion d'un produit de contraste iodé non-ionique), la phase lipidique peut comprendre en outre au moins une huile non iodée de densité inférieure à 1, préférentiellement une huile non iodée de densité inférieure à 0,96, encore plus préférentiellement une huile non iodée choisie parmi l'huile de lin, l'huile de soja, l'huile de palme, l'huile de coco, l'huile de ricin, l'huile de maïs, l'huile de coton, l'huile d'arachide, l'huile de sésame, l'huile de tournesol, l'huile de carthame, l'huile d'amande, l'huile d'olive, l'huile de pavot et une huile comprenant ou consistant en un mélange de triglycérides d'acide gras de formule : 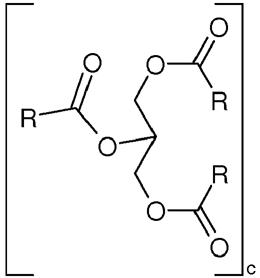 (R = C8 + C10) > 95 %
où R est une chaîne aliphatique comprenant de 3 à 35 atomes de carbone, sous réserve que plus de 95% desdits acides gras soient en C8 et/ou en C10, vendue par exemple sous le nom MIGLYOL®, par exemple l'huile MIGLYOL® 810, l'huile MIGLYOL® 812 (caprylic/capric triglyceride), l'huile MIGLYOL® 818 (caprylic/capric/linoleic triglyceride), l'huile MIGLYOL® 612 (glyceryl trihexanoate) ou d'autres dérivés MIGLYOL® propylène glycol dicaprylate dicaprate. L'expression (R = C8 + C10) > 95 % signifie que les triglycérides du mélange sont des triglycérides d'acides gras dont plus de 95% sont des acides gras en C8 et/ou en C10 (acide caprique ou caprylique). Lorsque l'acide gras est en C8, R est une chaine comprenant 7 atomes de carbone et lorsque l'acide gras est en C10, R est une chaine comprenant 9 atomes de carbone.

Les densités des différentes huiles non iodées listées sont précisées dans le tableau suivant :

| **Nom de l'huile** | **Densité** |
|---|---|
| Huile de lin | 0,94 |
| Huile de Soja | 0,92 |
| Huile de Miglyol® | 0,94 |
| Huile de palme | 0,90 |
| Huile de coco | 0,92 |
| Huile de ricin | 0,96 |
| Huile de maïs | 0,90 |
| Huile de coton | 0,92 |
| Huile d'arachide | 0,92 |
| Huile de sésame | 0,92 |
| Huile de tournesol | 0,93 |
| Huile de carthame | 0,92 |
| Huile d'amande | 0,91 |
| Huile d'olive | 0,915 |
| Huile de pavot | 0,928 |

Dans ce mode précis de réalisation, la densité de la phase lipidique comprenant l'huile iodée et une ou plusieurs huiles non iodées telles que définies ci-dessus est alors préférentiellement de 0,9 à 1,2, plus préférentiellement de 0,95 à 1,10, encore plus préférentiellement de 1,05.

La taille des gouttelettes de phase aqueuse est préférentiellement comprise de 1 à 200 µm, plus préférentiellement comprise de 5 à 100 µm, encore plus préférentiellement comprise de 5 à 50 µm, voire de 5 à 10 µm. Cette taille améliore encore d'avantage la stabilité de l'émulsion. La taille peut être mesurée au microscope optique (par exemple le microscope LEICA DM2000 LED).

Préférentiellement, les gouttelettes de phase aqueuse sont réparties de manière homogène. L'homogénéité est vérifiée à l'aide d'un microscope optique : si des agrégats de gouttelettes sont observés, ces gouttelettes ne sont pas réparties de manière homogène.

Le ratio volumique phase aqueuse / phase lipidique dans la composition sous forme d'émulsion selon l'invention est de manière avantageuse de 1/2 (soit 0,5) à 1/4 (soit 0,25), préférentiellement de 2/5 (soit 0,4) à 3/10 (soit 0,3), plus préférentiellement de 1/3 (soit environ 0,33). Un ratio inférieur à 1/2 permet d'obtenir de façon certaine une émulsion E/H. En effet, un ratio 1/1 entre la phase lipidique et la phase aqueuse favorise naturellement un sens H/E. Pour forcer le sens E/H, la quantité d'huile iodée ajoutée doit être augmentée. Au-delà d'un ratio 1/4, le risque d'embolie devient important. En effet, de façon à solubiliser une quantité thérapeutiquement efficace d'agent anticancéreux dans la phase aqueuse, il faut que cette phase aqueuse ait un volume suffisant. Avoir une phase lipidique comprenant une huile iodée et étant plus de 4 fois plus volumineuse que la phase aqueuse, a généralement pour conséquence que la dose d'huile iodée utilisée devient supérieure à la limite autorisée. Dans les mentions légales concernant un produit comme le Lipiodol®, il est indiqué que le volume injecté dans une procédure de radiologie interventionnelle ne doit pas dépasser 15 mL.

Les pourcentages volumiques de phases aqueuse et lipidique et le ratio volumique phase aqueuse / phase lipidique de la composition sous forme d'émulsion selon l'invention permettent d'obtenir systématiquement une émulsion inverse (E/H) qui permet d'améliorer l'acheminement d'un agent anticancéreux jusque dans une tumeur.

De manière avantageuse, la composition selon l'invention présente une viscosité à 20°C comprise de 100 à 200 mPa.s, préférentiellement comprise de 120 à 170 mPa.s, plus préférentiellement comprise de 150 à 165 mPa.s, et/ou une viscosité à 37°C comprise de 40 à 80 mPa.s, préférentiellement comprise de 50 à 70 mPa.s , plus préférentiellement comprise de 60 à 70 mPa.s. Les valeurs de viscosité sont obtenues à l'aide d'un rhéomètre Malvern Instruments Kinexus Pro, présentant une cellule cône-plan 4° avec un diamètre de 40 mm. Les mesures sont réalisées à contrainte imposée dans une gamme de 0,16 à 10 Pa.

### Les huiles iodées

On entend par le terme « acide gras » désigner des acides carboxyliques aliphatiques saturés ou insaturés présentant une chaîne carbonée d'au moins 4 atomes de carbone. Les acides gras naturels possèdent une chaîne carbonée de 4 à 28 atomes de carbone (généralement un nombre pair). On parle d' « acide gras à longue chaîne » pour une longueur de 14 à 22 carbones et à très longue chaîne s'il y a plus de 22 carbones. On parle au contraire d'« acide gras à courte chaîne » pour une longueur de 4 à 10 carbones, notamment 6 à 10 atomes de carbone, en particulier 8 ou 10 atomes de carbone. L'homme du métier connaît la nomenclature associée et en particulier utilise :
- Ci-Cp pour désigner une fourchette d'acides gras en Ci à Cp
- Ci+Cp, le total des acides gras en Ci et des acides gras en Cp

Par exemple :
- les acides gras de 14 à 18 atomes de carbone s'écrivent « acides gras en C14-C18 »
- le total des acides gras en C16 et des acides gras en C18 s'écrit C16 +C18.
- pour un acide gras saturé, un homme du métier utilisera la nomenclature suivante Ci : 0, où i est le nombre d'atomes de carbone de l'acide gras. L'acide palmitique sera par exemple désigné par la nomenclature (C16 :0).
- pour un acide gras insaturé, un homme du métier utilisera la nomenclature suivante Ci : x n-N où N sera la position de la double liaison dans l'acide gras insaturé en partant du carbone opposé au groupe acide, i est le nombre d'atomes de carbone de l'acide gras, x est le nombre de double liaisons (insaturations) de cet acide gras. L'acide oléique, sera par exemple désigné par la nomenclature (C18 :1 n-9).

De façon avantageuse, l'huile iodée selon l'invention comprend ou est constituée de dérivés d'acides gras iodés, préférentiellement d'esters éthyliques d'acides gras iodés, plus préférentiellement d'esters éthyliques d'acides gras iodés d'huile d'œillette, d'huile d'olive, d'huile de graines de colza, d'huile d'arachide, d'huile de graines de soja ou d'huile de noix, encore plus préférentiellement d'esters éthyliques d'acides gras iodés d'huile d'œillette ou d'huile d'olive. Plus préférentiellement, l'huile iodée selon l'invention comprend ou est constituée d'esters éthyliques d'acides gras iodés d'huile d'œillette (aussi appelée pavot noir ou *Papaver somniferum var. nigrum*)*.* L'huile d'œillette, aussi appelée huile de graines de pavot ou huile de graines d'œillette, contient préférentiellement plus de 80% d'acides gras insaturés (en particulier de l'acide linoléique (C18 :2 n-6) et de l'acide oléique (C18 :1 n-9)) dont au moins 70% d'acide linoléique et au moins 10% d'acide oléique. L'huile iodée est obtenue à partir de la complète iodation d'une huile telle que l'huile d'œillette dans des conditions permettant une liaison d'un atome d'iode pour chaque double liaison des acides gras insaturés (Wolff et al. 2001, Medicine 80, 20-36) suivie d'une trans-estérification.

L'huile iodée selon l'invention contient préférentiellement de 29 à 53% (m/m), plus préférentiellement 37% à 39% (m/m) d'iode.

Comme exemples d'huiles iodées peuvent être cités le Lipiodol®, le Brassiodol® (issu de l'huile de graines de colza (*Brassica compestis*)*,* le Yodiol® (issu de l'huile d'arachide), l'Oriodol® (issu de l'huile d'œillette mais sous forme de triglycérides d'acides gras), le Duroliopaque®(issu de l'huile d'olive).

Préférentiellement, l'huile iodée est le Lipiodol®, une huile iodée utilisée comme produit de contraste et dans certaines procédures de radiologie interventionnelle. Cette huile est un mélange d'esters éthyliques d'acides gras iodés et non-iodés d'huile de graines d'œillette. Elle consiste majoritairement (en particulier, plus de 84%) en un mélange d'esters éthyliques d'acides gras iodés à longue-chaine (en particulier des acides gras en C18) issus de l'huile de graines d'œillette, préférentiellement en un mélange de monoiodostéarate d'éthyle et de diiodostéarate d'éthyle. L'huile iodée peut aussi être une huile à base d'ester éthylique monoiodé d'acide stéarique (C18 :0) issu de l'huile d'olive. Un produit de ce type, s'appelant Duroliopaque® était commercialisé il y a quelques années.

Les caractéristiques principales de Lipiodol® sont les suivantes :

| **Composés** | **Proportions dans le mélange d'acide gras** |
|---|---|
| Palmitate d'éthyle (Ethyl C16 :0) | 4,6 à 6,7% (m/m), préférentiellement 4,8% (m/m) |
| Stéarate d'éthyle (Ethyl C18 :0) | 0,8 à 1,9% (m/m), préférentiellement 1,2% (m/m) |
| Monoiodostéarate d'éthyle | 11,3 à 15,3% (m/m), préférentiellement 13,4% (m/m) |
| Diiodostéarate d'éthyle | 73,5 à 82,8% (m/m), préférentiellement 78,5% (m/m) |

| **Autres caractéristiques du Lipiodol®** : | |
|---|---|
| Iode | 37% à 39% (m/m) (soit 480 mg/ml) |
| Viscosité | |
| à 37°C | 25 mPa.s |
| à 20°C | 50 mPa.s |
| Densité | 1,268 - 1,290 g/cm³ à 20°C, préférentiellement 1,28 |

De manière préférentielle, la quantité d'huile iodée présente dans la composition selon l'invention ne dépasse pas 15 mL.

Préférentiellement, la phase lipidique est constituée essentiellement d'huile iodée telle que définie ci-dessus et d'un tensioactif de formule (I) ou (I'). Dans un mode de réalisation particulier de l'invention, la phase lipidique est constituée essentiellement d'huile iodée telle que définie ci-dessus, d'une huile non iodée telle que définie ci-dessus et d'un tensioactif de formule (I) ou (I').

### Agent anticancéreux

L'agent anticancéreux vectorisé par la composition selon l'invention ou compris dans la composition sous forme d'émulsion selon l'invention est préférentiellement choisi parmi les anthracyclines, les complexes de platine, les composés apparentés aux anthracyclines tels que la mitoxantrone et la nemorubicine, les antibiotiques tels que la mitomycine C (Ametycine®), la bléomycine et l'actinomycine D, les autres composés anti-néoplasiques tels que l'irinotecan, le 5-Fluoro-Uracile (Adrucil®), le sorafénib (Nevaxar®), le sunitinib (Sutent®), le regorafénib, le brivanib, l'orantinib, le linsitinib, l'erlotinib, le cabozantinib, le foretinib, le tivantinib, la fotémustine, la tauromustine (TCNU), la carmustine, la cytosine C, le cyclophosphonamide, la cytosine arabinoside (ou cytarabine), le paclitaxel, le docétaxel, le methotrexate, l'everolimus (Afinitor®), le PEG-arginine deiminase, la combinaison tegafur/gimeracil/oteracil (Teysuno®), le muparfostat, le pérétinoine, la gemcitabine, le bévacizumab (Avastin®), le ramucirumab, la floxuridine, les immunostimulants tels que le GM-CSF (Granulocyte-macrophage colony-stimulating factor) et ses formes recombinantes : molgramostim ou sargramostim (Leukine®), l'OK-432 (Picibanil®), l'interleukine-2, l'interleukine-4 et le Tumor necrosing Factor-alpha (TNFalpha), les anticorps anti-CEA (CarcinoEmbryonic Antigen) marqués à l'¹²⁵l, les microsphères chargées en l'un des composés cités précédemment, les radio-éléments, les complexes de ces radioéléments avec des chélates, les particules magnétiques à base d'un composé de fer (« ultrasmall superparamagnetic particles of iron oxide » ou USPIOs) et/ou d'un chélate de gadolinium, les microsphères radioactives, les séquences d'acide nucléique et un mélange d'un ou plusieurs de ces composés (préférentiellement un mélange d'une ou plusieurs anthracyclines ou un mélange d'une anthracycline et d'un radioélément tels que cités ci-dessus ou un mélange d'une anthracycline et d'une particule à base d'un composé de fer) et/ou d'un chélate de gadolinium.

Préférentiellement, la phase aqueuse de la composition selon l'invention comprend 0,5% à 2,5% (m/v), plus préférentiellement 1 à 2% (m/v) d'agent anticancéreux dans la phase aqueuse.

La composition sous forme d'émulsion peut comprendre un ou plusieurs agents anticancéreux. De préférence, au moins un agent anticancéreux est hydrosoluble, c'est-à-dire qu'il est soluble à plus de 50% dans la phase aqueuse. Aussi, lorsque la composition sous forme d'émulsion ne comprend qu'un agent anticancéreux, celui-ci est de préférence hydrosoluble et se situe donc dans la phase aqueuse dispersée. Lorsque la composition sous forme d'émulsion comprend plusieurs agents anticancéreux, certains d'entre eux peuvent se situer dans la phase lipidique continue.

L'agent anticancéreux préférentiel est choisi parmi les anthracyclines, la mitomycine C, les complexes de platine, les radioéléments et les complexes de ceux-ci listés ci-dessus. L'agent anticancéreux est choisi plus préférentiellement parmi les anthracyclines et encore plus préférentiellement parmi la doxorubicine, l'épirubicine, la némorubicine et l'idarubicine.

De manière avantageuse, l'agent anticancéreux est choisi parmi les agents intercalants tels que la doxorubicine, l'épirubicine, l'idarubicine, la némorubicine, le mitoxantrone et la pirarubicine ; les agents alkylants tels que le cisplatine, le carboplatine, l'oxaliplatine, la lobaplatine, le cyclophosphonamide et la mitomycine C, la fotémustine ; les inhibiteurs de topo-isomérase de type 1 tels que l'irinotécan ; les inhibiteurs de topo-isomérase de type 2 tels que la doxorubicine et le mitoxantrone ; les inhibiteurs de tyrosine kinases tels que l'everolimus ; les inhibiteurs multikinases tels que le sorafénib , les agents antimétabolites tels que le 5-Fluoro-Uracile, le méthotrexate et la gemcitabine, les radioéléments tels que listés ci-dessus, les complexes de ces radioéléments avec des chélates macrocycliques, les particules magnétiques à base d'un composé de fer, les microsphères radioactives, les séquences d'acide nucléique et un mélange de ceux-ci.

Préférentiellement, les anthracyclines évoquées ci-dessus sont choisies parmi la doxorubicine (ou adriamycine vendue sous le nom Adriblastine® par Pfizer), l'épirubicine (Farmorubicine®), l'idarubicine (Zavedos®), la daunorubicine, la pirarubicine, la némorubicine et le mélange d'un ou plusieurs de ces composés.

Préférentiellement, les complexes de platine évoqués ci-dessus sont choisis parmi le cisplatine (Platinol AQ®), le carboplatine, le miriplatine, l'oxaliplatine (Eloxatine®), la lobaplatine et le mélange d'un ou plusieurs de ces composés.

Préférentiellement, les radio-éléments évoqués ci-dessus sont choisis parmi le Rhénium 186 (¹⁸⁶Re), le Rhénium 188 (¹⁸⁸Re), l'Yttrium 90 (⁹⁰Y), le Lutétium 177 (¹⁷⁷Lu), l'Holmium 166 (¹⁶⁶Ho), l'Iode 125 (¹²⁵I), l'Iode 131 (¹³¹I), le Phosphore 32 (³²P), le Strontium 89 (⁸⁹Sr), le Samarium 153 (¹⁵³Sm), le Cuivre 67 (⁶⁷Cu), l'Etain 117m (^{117m}Sn), le Bismuth 213 (²¹³Bi), le Bismuth 212 (²¹²Bi), l'Astate 211 (²¹¹At), le Radium 223 (²²³Ra), l'indium 111 (¹¹¹In), le Gallium 67 (⁶⁷Ga), le Gallium 68 (⁶⁸Ga), le Technétium 99 métastable (99mTc) et un mélange d'un ou plusieurs de ces composés. Le radioélément, éventuellement sous forme complexée avec des chélates linéaires ou macrocycliques, est choisi préférentiellement parmi ¹⁸⁸Re, ⁹⁰Y, ¹⁷⁷Lu, ¹⁶⁶Ho, ¹³¹I, ¹¹¹In, ⁶⁷Ga, ⁶⁸Ga et 99mTc ou encore plus préférentiellement parmi ¹⁸⁸Re, ⁹⁰Y, ¹⁷⁷Lu, ¹⁶⁶Ho, et ¹³¹I. Préférentiellement, les chélates des complexes de ces radioéléments évoqués ci-dessus sont choisis parmi les chélates linéaires et les chélates macrocycliques tels que le DOTA, le PCTA, le DTPA, le NOTA et leurs dérivés, plus préférentiellement parmi les chélates macrocycliques tels que le DOTA, le PCTA, le NOTA et leurs dérivés. L'Yttrium 90 (⁹⁰Y) et les complexes d'Yttrium 90 et de chélates macrocycliques tels que définis ci-dessus sont des composés préférentiels dans leurs catégories respectives.

Préférentiellement, les séquences d'acide nucléique évoquées ci-dessus sont choisies parmi des séquences d'acide désoxyribonucléique (ADN) et d'acide ribonucléique (ARN), plus préférentiellement choisies parmi des séquences d'ADN ou d'ARN vectorisées par des vecteurs de thérapie génique tels que des vecteurs viraux choisis parmi les vecteurs adénoviraux (virus à ADN), les vecteurs rétroviraux (virus à ARN), les vecteurs dérivés de virus adéno-associés ou AAV (Adeno Associated Virus) et les vecteurs dérivés d'autres virus (tels que les virus Herpes Simplex (HSV), les poxvirus, les virus de la grippe) et des vecteurs non viraux tels que des polycations ou des nanoparticules (en particulier d'hydroxyapatite ou d'hydroxyapatite modifiée (telle que la poly-L-lysine (PLL)-modified hydroxyapatite)) et des séquences d'ARN interférant (pARNi ou siRNA pour « small interfering RNA ») ou d'ARN double brin (dsRNA).

Les séquences d'acide nucléique sont préférentiellement choisies parmi les séquences natives ou modifiées ou une partie des séquences natives ou modifiées du gène codant pour la protéine p53, pour la protéine Rb (en particulier le gène Rb1) ou pour le gène codant pour l'interleukine 12 (IL-12) ou leurs transcrits respectifs (i.e sous forme d'ARN).

La forme commerciale de ces agents anticancéreux est le plus souvent la forme lyophilisée ou la forme pulvérisée (i.e. sous forme de poudre). Ces lyophilisats ou poudres d'agents anticancéreux peuvent contenir les excipients classiquement utilisés dans le domaine pharmaceutique : lactose (agent solubilisant et lyophilisant), parahydrobenzoate de méthyle (antioxydant) et/ou chlorure de sodium (NaCl).

Par "particules à base d'un composé du fer", on entend, au sens de la présente description, des particules comprenant ou constituées d'un composé du fer, comprenant généralement du fer (III), généralement un oxyde ou un hydroxyde de fer. On parle souvent d'Ultra Small Particles of Iron Oxide ou USPIOs.

En règle générale, les particules magnétiques sont composées en tout ou partie d'hydroxyde de fer ; d'oxyde de fer hydraté ; de ferrites ; d'oxydes de fer mixtes tels que des oxydes de fer mixtes de cobalt, de nickel, de manganèse, de béryllium, de magnésium, de calcium, de baryum, de strontium, de cuivre, de zinc ou de platine ; ou d'un mélange de ceux-ci.

Selon une variante particulièrement préférée, les particules magnétiques sont superparamagnétiques.

Les particules magnétiques avant d'être recouvertes par le revêtement approprié possèdent alors, de préférence, un diamètre cristallin de 5 à 200 nm, mieux encore de 10 à 60 nm ou de 10 à 20 nm.

Dans un mode de réalisation avantageux, les particules magnétiques à base d'un composé du fer, sont recouvertes par un composé hydrophile, préférentiellement de type Polyéthylène glycol (PEG), plus préférentiellement un PEG de masse molaire comprise de 1500 à 3000.

Dans un autre mode de réalisation avantageux, les particules magnétiques à base d'un composé du fer, sont recouvertes par un acide gras insaturé, préférentiellement mono-insaturé, encore plus préférentiellement par de l'acide oléique (C 18:1 n-9). Les particules magnétiques ainsi rendues liposolubles sont suspendues dans la phase lipidique continue.

Au sens de la présente demande, le terme de "ferrite" désigne les oxydes de fer de formule générale [x Fe₂O₃, y MO_{z}], où M désigne un métal magnétisable sous l'effet d'un champ magnétique tel que Fe, Co, Ru, Mg, Mn, le métal magnétisable pouvant être éventuellement radioactif.

De façon préférentielle, les particules magnétiques des compositions de l'invention comprennent une ferrite, notamment la maghémite (γ Fe₂O₃) ou la magnétite (Fe₃O₄), ou encore les ferrites mixtes de cobalt (Fe₂CoO₄) ou de manganèse (Fe₂MnO₄). Dans ce cadre, on préfère tout particulièrement les particules magnétiques composées en tout ou partie d'une ferrite, et de préférence essentiellement (c'est-à-dire plus de 90% préférentiellement plus de 95%, encore plus préférentiellement plus de 98% en poids), de maghémite ou de magnétite ou d'un mélange de ceux-ci.

Préférentiellement, les microsphères radioactives évoquées ci-dessus sont constituées d'une résine échangeuse de cations (comprenant par exemple un alcool polyvinylique ou un copolymère comprenant du styrène et du divinyl benzene comme l'Aminex 50W-X4 de la société Biorad) marquée à l'Yttrium 90 (SIR-Spheres® commercialisées par la société SIRTeX Medical Ltd) ou sont constituées de verre dans lequel de l'Yttrium 90 a été incorporé (TheraSphere® commercialisées par la société BTG) ou sont constituées d'un polymère tel que l'acide polylactique (PLLA) et de l'un des radioéléments évoqués ci-dessus, l'holmium (166Ho) étant alors le radioélément préféré. Plus préférentiellement, c'est de l'Yttrium sous forme de ⁸⁹Y₂O₃ qui est incorporé dans les microsphères constituées de verre, celles-ci étant ensuite irradiées par des neutrons pour les rendre radioactives en transformant l'Yttrium froid ⁸⁹Y en Yttrium radioactif ⁹⁰Y. Encore plus préférentiellement, les microsphères constituées d'une résine échangeuse de cations ou constituées de verre ont respectivement un diamètre de 20 à 60 µm et de 20 à 30 µm. Les microsphères du type SIR-Spheres ont fait notamment l'objet du brevet EP 0 740 581 B1.

Préférentiellement, les microsphères chargées en l'un des composés cités précédemment, sont chargées en une anthracycline telle que la doxorubicine, l'épirubicine ou l'idarubicine ou en un inhibiteur de topo-isomérase de type I tel que l'irinotecan ou en un complexe de platine comme le cisplatine. Ces microsphères sont préférentiellement produites à partir d'alcool polyvinylique (PVA). De manière préférentielle, elles sont constituées d'un hydrogel de PVA et sont encore plus préférentiellement constituées d'un polymère de PVA modifié par des groupements sulfonates SO3⁻ sur lesquels viennent se fixer les composés cités ci-dessus quand ils sont chargés positivement (DC Beads®, DC-Beads M1® et LC-Beads® vendues par la société Biocompatibles) ou elles sont produites à partir de monomères tels que l'acétate de vinyle et l'acrylate de méthyle qui, quand ils sont combinés ensemble, forment un copolymère PVA / acrylique (copolymère de poly (acrylate de sodium - co-vinyl alcool) modifié par des groupements carboxylates COO- sur lesquels viennent se fixer, par simple liaison ionique, les composés cités ci-dessus quand ils sont chargés positivement (Hepasphere® ou Quadrasphere® vendues par Merit Medical). Ces microsphères peuvent également être constituées de polymère de polyphosphazène et sont alors chargées en doxorubine, en épirubicine, en idarubicine ou en irinotecan (microsphères Embozene Tandem® vendues par la société Celonova Biosciences). Elles peuvent aussi être constituées d'un polymère obtenu à partir de fécule de pomme de terre hydrolysée, réticulée et substituée avec des groupements d'éther de glycérol et sont alors chargées en doxorubicine, actinomycine D, tauromustine, cisplatine, carboplatine, mitomycine C, fotémustine, carmustine, irinotecan, 5-FU, floxuridine, docetaxel, en des anticorps anti-CEA (CarcinoEmbryonic Antigen) marqués à l'¹²⁵I ou en complexe 99mTc-DTPA (Microsphères Embocept® S vendues par Pharmacept).

### Agent tensioactif

On rappelle que le terme « tensioactif » ou « surfactant » fait référence à un composé à structure amphiphile qui lui confère une affinité particulière pour les interfaces de type eau/huile ce qui lui donne la capacité d'abaisser l'énergie libre de ces interfaces et de stabiliser des systèmes dispersés.

La composition selon l'invention comprend au moins un tensioactif de formule (I) ou (I') telle que définie ci-dessus. Elle peut donc comprendre un tensioactif de formule (I) ou (I') ou un mélange de tensioactifs de formule (I) ou (I').

Le tensioactif a la formule (I) ou (I') telle que définie ci-dessus, de préférence, dans laquelle s vaut 0 ou 1, m représente un nombre entier de 2 à 10 et R₁ représente un groupement de formule (II) telle que définie ci-dessus, dans laquelle n représente un nombre entier de 5 à 7, o représente un nombre entier de 1 à 3, p représente un nombre entier de 3 à 5, q représente un nombre entier de 2 à 5 et r vaut 0 ou 1. De manière encore plus préférentielle, dans la formule (I) ou (I') telle que définie ci-dessus, s vaut 1, m représente un nombre entier de 2 à 5 et n vaut 7, o vaut 1, p vaut 5 et q représente un nombre entier de 2 à 4 et r vaut 1 dans la formule (II) que représente R₁.

La HLB (signifiant Hydrophilic-Lipophilic Balance, ou équilibre hydrophile/lipophile) est une grandeur, bien connue de l'homme du métier, caractéristique d'un tensioactif. De manière préférentielle, le tensioactif selon l'invention est un tensioactif à faible HLB c'est-à-dire un tensioactif ayant une valeur de HLB comprise de 1 à 8, préférentiellement comprise de 1 à 6. Le HLB permet de déterminer le type d'émulsion huile dans l'eau ou eau dans l'huile, comme illustré dans l'article de W.C. Griffin (« classification of Surface-active agents by « HLB » », Journal of the Society of Cosmetic Chemists, 1949, 311-326). Cet article indique en particulier que pour des tensioactifs dont les HLB sont de 4 à 6, des émulsions de type E/H sont observées, alors que pour des tensioactifs dont les HLB sont de 8 à 18, des émulsions de type H/E sont plutôt observées.

De manière avantageuse, le tensioactif de formule (I) ou (I') est soluble dans l'huile iodée et cela, en particulier dans les gammes de proportions indiquées ci-dessus.

De manière avantageuse, le tensioactif de formule (I) ou (I') selon l'invention est choisi parmi le polyricinoléate de polyglycérol et le PEG-30-dipolyhydroxystéarate.

Le polyricinoléate de polyglycérol ou PGPR (Palsgaard®4125, Palsgaard®4150, Palsgaard®4110, Palsgaard®4120 ou Palsgaard®4175) est un tensioactif ayant comme groupe hydrophile du poly-glycérol (préférentiellement constitué d'au moins 75% de di et tri-glycérol et d'au maximum 10% d'heptaglycerol) et comme groupe hydrophobe des acides gras ricinoléiques interestérifiés. Il a une HLB de 1,5.

Il correspond à un tensioactif de formule I, telle que définie ci-dessus, dans laquelle :
- s vaut 1,
- m représente un nombre entier de 2 à 5,
- R₁ représente un groupement de formule (II) telle que définie ci-dessus dans laquelle n vaut 7, o vaut 1, p vaut 5, q vaut 2 à 4 et r vaut 1,
- R₂ représente R₁ et/ou un atome d'hydrogène.

De manière préférentielle, le tensioactif de formule (I) ou (I') est un mélange de tensioactifs de formule (I) ou (I') dans laquelle :
- s vaut 1,
- m vaut 2, 3, 4 ou 5,
- R₁ représente un groupement de formule (II) telle que définie ci-dessus dans laquelle n vaut 7, o vaut 1, p vaut 5, q vaut 2, 3 ou 4 et r vaut 1,
- R₂ représente R₁ et/ou un atome d'hydrogène.

De manière préférentielle, le tensioactif de formule (I) ou (I') selon l'invention est un mélange de tensioactifs choisi parmi les composés de formule : 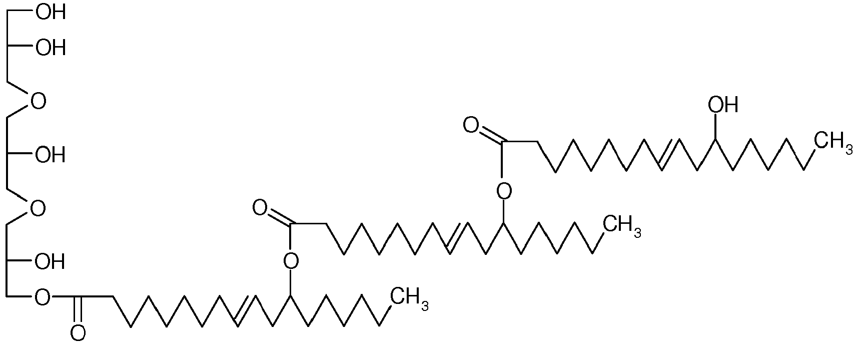 ou 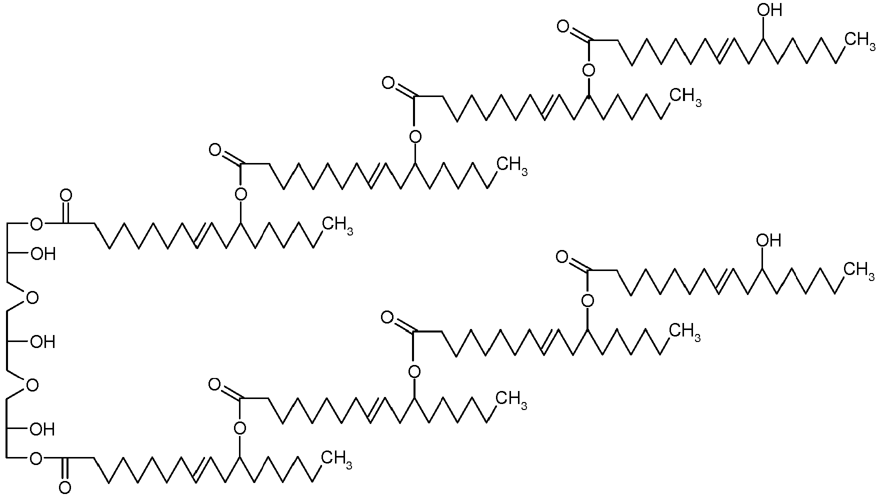 et

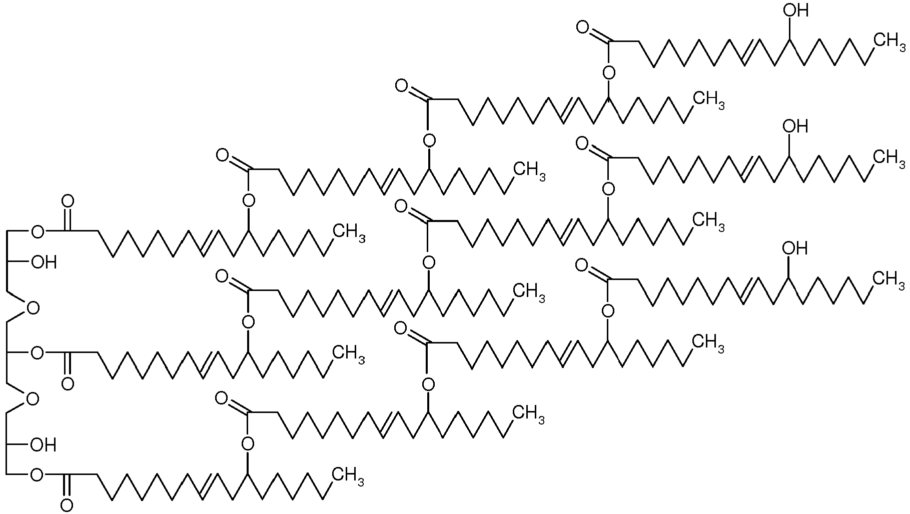

Le PEG-30-dipolyhydroxystéarate (Cithrol® DPHS et anciennement Arlacel® P135 vendu par la société Croda) a une HLB de 5-6. La dénomination PEG est conforme aux conventions de nomenclature fixées par l'INCI, la valeur 30 précisée ci-dessus correspondant au nombre moyen d'unités monomériques d'oxyde d'éthylène.

Il correspond à un tensioactif de formule I, telle que définie ci-dessus, dans laquelle :
- s vaut 0,
- m vaut 30,
- R₁ représente un groupement de formule (II) telle que définie ci-dessus dans laquelle n vaut 9, o vaut 1, p vaut 5, q vaut 7 et r vaut 0,
- R₂ est identique à R₁.

### Utilisation de la composition selon l'invention

Selon un second objet, l'invention porte sur l'utilisation de la composition telle que définie ci-dessus pour vectoriser un agent anticancéreux. L'invention concerne également une composition sous forme d'émulsion telle que définie ci-dessus, pour son utilisation dans le traitement du cancer ou de ses métastases, préférentiellement par chimioembolisation intra-artérielle. Dans un mode de réalisation avantageux, l'invention porte sur l'utilisation d'une composition selon l'invention pour la préparation d'un médicament pour le traitement du cancer ou de ses métastases, préférentiellement par chimioembolisation intra-artérielle. La chimioembolisation intra-artérielle est définie comme l'introduction percutanée par voie intra-artérielle d'une substance pour obstruer un vaisseau sanguin en combinaison avec un agent anticancéreux, afin de délivrer une quantité thérapeutiquement efficace de cet agent dans une tumeur. Préférentiellement, le cancer ainsi traité est choisi parmi le cancer du foie (en particulier le cancer primitif du foie comme le carcinome hépatocellulaire ou CHC), le cholangiocarninome, les métastases hépatiques des cancers primaires choisis parmi le cancer colorectal, les tumeurs neuroendocrines, les cancers du sein, les cancers du rein et les mélanomes.

La chimioembolisation d'une tumeur hépatique est réalisée, de façon préférentielle, par la mise en œuvre des étapes successives suivantes :
a) cathétérisme percutané à partir de l'artère fémorale,
b) administration de l'émulsion selon l'invention jusqu'à ce que soit observée une stase dans les branches de second ou de troisième ordre,
c) optionnellement, l'administration d'un agent embolisant dans la tumeur après que l'émulsion ait été administrée.

De manière préférentielle, l'émulsion selon l'invention ainsi administrée ne comprend pas plus de 20 mL d'huile iodée, plus préférentiellement pas plus de 15 ml d'huile iodée.

Le cathétérisme, qui consiste à amener un tube, appelé cathéter, jusque dans l'artère hépatique puis dans la branche de cette artère qui perfuse la lésion cancéreuse, est avantageusement réalisé avec l'assistance d'une technique d'imagerie. Des logiciels de guidage sont d'ailleurs proposés aux radiologues interventionnels pour leur permettre de placer leur cathéter de la manière la plus optimale possible.

Par « agent embolisant », on entend un ou plusieurs composés permettant de ralentir ou de stopper définitivement ou temporairement le flux sanguin dans un vaisseau. Comme exemple d' « agent embolisant », on peut citer l'éponge de gélatine, les particules de mousse de gélatine (Geifoam®, Spongel®, Curaspon®), l'alcool polyvinylique (PVA) ou des microsphères calibrées à base par exemple de trisacrylgelatine, de PVA (Ivalon®, Contour®), etc...

De manière avantageuse, avant la procédure de chimioembolisation, une angiographie ou artériographie, réalisée à l'aide d'un angioscanner ou d'un angio MR (Angiographie par Résonance Magnétique ou MRA) et le plus souvent une injection de produit de contraste (par exemple, pour l'angioscanner : des produits de contraste iodés hydrosolubles comme de l'iobitridol (Xenetix®) ou de l'iohexol (Omnipaque®), et pour l'angioMR : des chélates de gadolinium comme l'acide gadotérique (Dotarem®) ou le gadobutrol (Gadovist®)), est pratiquée afin de repérer la vascularisation viscérale et la perfusion artérielle de la (ou des) tumeur(s).

Cette technique de chimioembolisation peut être utilisée seule ou en combinaison avec une ou plusieurs autres techniques évoquées ci-dessous. On peut également la substituer à l'une de ces autres techniques.

Dans le cas où l'agent anticancéreux est choisi parmi des radioéléments ou des complexes de radioéléments avec des chélates macrocycliques évoqués ci-dessus, la technique utilisée est la radiothérapie sélective interne ou radioembolisation. Elle consiste à injecter la composition selon l'invention directement dans la branche de l'artère hépatique qui perfuse la tumeur. Cette technique a comme avantage de délivrer une irradiation très importante à la tumeur sans pour autant irradier de façon importante le foie sain et les autres organes du patient.

Dans le cas où l'agent anticancéreux est choisi parmi des particules magnétiques à base d'un composé de fer (USPIOs), la technique utilisée est l'hyperthermie magnétique ablative. Celle-ci consiste à induire une élévation locale de la température au niveau du tissu tumoral, les cellules tumorales étant plus sensibles à une élévation de température que des cellules saines. Cette élévation de la température est provoquée par l'utilisation d'un stimulus externe et en particulier l'application d'un champ magnétique alternatif à la zone que l'on souhaite traiter. On distingue deux types d'hyperthermie selon la température atteinte : pour des températures supérieures à 46°C, il est possible d'induire une nécrose tissulaire et on parle alors de thermoablation ; des températures de 42°C à 46°C altèrent les fonctions de nombreuses protéines structurelles et enzymatiques, modifiant le développement et la différenciation cellulaire et pouvant induire l'apoptose, on parle alors d'hyperthermie modérée. Si les cellules ne meurent pas, elles deviennent plus sensibles aux rayons ionisants ou à la chimiothérapie.

Dans le cas où l'agent anticancéreux est une séquence d'acide nucléique choisie parmi des séquences d'acide désoxyribonucléique (ADN) et d'acide ribonucléique (ARN) vectorisé par un vecteur viral ou un vecteur non viral tel qu'évoqué ci-dessus ou une séquence d'ARN interférant (pARNi ou siRNA pour « small interfering RNA ») ou d'ARN double brin (dsRNA), la technique utilisée est la thérapie génique, parfois aussi appelée « génothérapie ». Le principe de cette approche est d'introduire un gène étranger dont le produit d'expression induit (directement ou directement) la mort des cellules tumorales. Schématiquement, trois approches sont utilisables : a) l'induction d'une défense immunitaire (« immunostimulation ») en modifiant les antigènes de la membrane des cellules tumorales ; b) le transfert d'un gène dit « suppresseur » de tumeur dans le génome de la cellule tumorale, ou enfin c) le transfert d'un gène dit « suicide » qui permet de transformer une pro-drogue d'agent anticancéreux non active en une molécule toxique pour les cellules tumorales.

Toutes ces différentes techniques sont connues de l'homme du métier. Ce dernier saura aisément choisir les paramètres à ajuster pour mettre en œuvre ces techniques en utilisant la composition selon l'invention.

Par les termes « traiter » et « traitement », sauf indication contraire, on entend toute action visant à améliorer le confort, le bien-être et la survie d'un individu, ce terme couvre donc aussi bien atténuer, diminuer, soulager que curer.

### Préparation de la composition sous forme d'émulsion selon l'invention

La composition sous forme d'émulsion est préférentiellement préparée extemporanément.

L'invention porte aussi sur un procédé de préparation d'une composition sous forme d'émulsion telle que définie ci-dessus, comprenant les étapes suivantes :
a) Mélange du tensioactif de formule (I) ou (I') telle que définie plus haut dans l'huile iodée, et
b) Mélange de la solution obtenue à l'étape a) avec une solution aqueuse comprenant un agent anticancéreux et un agent densifiant choisi parmi les complexes de chélate macrocyclique non-ionique avec un métal paramagnétique décrits ci-dessus.

La phase lipidique préparée à l'étape a) peut en outre comprendre une huile non-iodée telle que définie ci-dessus.

Le mélange effectué à l'étape b) peut être effectué par tout moyen connu de l'homme du métier. Préférentiellement, un robinet trois voies est utilisé. L'huile iodée comprenant le tensioactif est mise dans une première seringue qui est fixée sur le robinet trois voies. La solution aqueuse comprenant l'agent anticancéreux est mise dans une seconde seringue fixée également à ce robinet trois voies à 90°.

En appuyant alternativement sur les pistons des deux seringues (préférentiellement de 20 à 35 fois), un mélange des deux phases est effectué. Préférentiellement, on effectue un passage de l'intégralité du mélange dans une seringue puis dans l'autre toutes les 1 à 2 secondes. La troisième voie du robinet permet de fixer un cathéter avancé de façon sélective, sous contrôle fluoroscopique, jusqu'à la lésion tumorale, pour administration de l'émulsion.

De manière préférentielle, la préparation de la composition selon l'invention est effectuée à une température entre 10 et 40°C, plus préférentiellement entre 20 et 30°C.

### Formes de commercialisation de la composition selon l'invention

L'invention concerne également un kit comprenant :
- un tensioactif de formule (I) ou (I') telle que définie ci-dessus,
- une huile iodée,
- un agent anticancéreux
- un agent densifiant choisi parmi les complexes de chélate macrocyclique non-ionique avec un métal paramagnétique évoqués ci-dessus,
   comme produits de combinaison pour une utilisation simultanée, séparée ou étalée dans le temps pour son utilisation pour le traitement du cancer.

Le tensioactif, l'huile iodée, l'agent anticancéreux (ce dernier composé étant généralement sous forme de poudre) et l'agent densifiant (ce dernier composé étant généralement sous forme liquide) sont dans trois contenants différents, Un premier contenant comprend l'huile iodée dans laquelle le tensioactif de formule (I) ou (I') telle que définie ci-dessus a été ajouté, Un second contenant comprend l'agent anticancéreux sous forme de poudre. Un troisième contenant comprend une solution comprenant l'agent densifiant ou le mélange d'agent densifiant évoqué ci-dessus. Pour l'utilisation de ce kit, l,'agent anticancéreux sous forme de poudre est solubilisé dans la solution comprenant l'agent densifiant. Généralement, le mélange des [tensioactif/huile iodée et agent densifiant/agent anticancéreux en solution aqueuse] conduit à la composition sous forme d'émulsion selon l'invention.

De plus, l'invention concerne un kit comprenant :
- une composition comprenant le tensioactif de formule (I) ou (I') telle que définie ci-dessus et une huile iodée,
- une composition comprenant un agent anticancéreux et un agent densifiant choisi parmi les complexes de chélate macrocyclique non-ionique avec un métal paramagnétique,
   comme produits de combinaison pour une utilisation simultanée, séparée ou étalée dans le temps pour son utilisation pour le traitement du cancer.

La composition comprenant le tensioactif de formule (I) ou (I') telle que définie ci-dessus et une huile iodée et la composition comprenant l'agent anticancéreux et l'agent densifiant évoqué ci-dessus (présentés préférentiellement sous forme liquide) sont dans deux contenants différents. De manière préférentielle, l'agent anticancéreux et l'agent densifiant évoqué ci-dessus sont solubilisés extemporanément ou la veille de l'intervention dans une solution aqueuse. De préférence, la composition consiste en un mélange de tensioactif de formule (I) ou (I'), d'une huile iodée et éventuellement d'une huile non iodée. Généralement, le mélange de la composition, de l'agent anticancéreux et de l'agent densifiant évoqué ci-dessus en solution aqueuse conduit à la composition sous forme d'émulsion selon l'invention.

Par « contenant », on entend désigner tout récipient pharmaceutiquement acceptable pouvant contenir un produit. A titre d'exemple, on peut citer une ampoule, un flacon, une seringue pré-remplie.

Par « récipient pharmaceutiquement acceptable », on entend désigner tout récipient n'interagissant pas avec le produit, préférentiellement tout récipient ne libérant pas de composés dans l'huile iodée et ne dégradant pas l'huile iodée.

Les exemples figurant ci-après sont présentés à titre illustratif et non limitatif de l'invention.

### Exemple 1 :

### 1. Préparation de compositions sous forme d'émulsion selon l'invention :

### 1.1. Emulsions de Lipiodol® et d'anthracycline

50 mg de doxorubicine (Actavis) ont été reconstitués dans 2,5 mL de gadobutrol (aussi appelé Gd-BT-DO3A et vendu sous le nom commercial Gadovist® 1mmol/mL par la société Bayer). Après agitation manuelle pendant 30 secondes pour une bonne dissolution, la solution obtenue a été prélevée avec une seringue de 20mL luer lock. Cette seringue a alors été placée sur un robinet trois voies.

Le PGPR (1% m/v total, 100 mg - Interchim) a été dissout dans 7,5 ml de Lipiodol® par agitation manuelle.

L'huile obtenue a été prélevée avec une seringue luer lock de 20 mL qui a aussi été placée sur le robinet trois voies à 90°C. 34 passages, soit 17 aller-retours à force moyenne ont été réalisés en commençant par l'eau dans l'huile.

Pour ces émulsions, les volumes de la phase aqueuse et de phase lipidique choisis étaient respectivement de 2,5 mL (soit 25% v/v) et 7,5 mL (soit 75% v/v). Le ratio phase aqueuse / phase lipidique était de 1/3.

D'autres émulsions ont été réalisées :
- en ne mettant pas d'agent densifiant, ou
- en remplaçant l'agent densifiant Gadovist® par le complexe de chélate macrocyclique non-ionique avec un ion de gadolinium de formule suivante 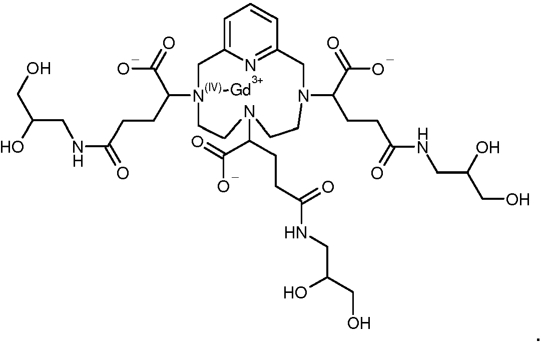 (appelé composé X ci-après) ou en le remplaçant avec un agent densifiant choisi parmi les produits de contraste iodés (du Xenetix® 250, du Xenetix® 300 (300 mg d'iode/mL), du Iopamiron® 350, du Iopamiron® 300, du Iomeron® 300, de l'Ultravist® 300 ou de l'Omnipaque® 240).
- en remplaçant l'agent densifiant Gadovist® par un mélange composé à 30% en volume de ProHance® et à 70% en volume de Xenetix® 350.
- en remplaçant le tensioactif PGPR par le Cithrol™ DPHS (PEG-30 Dipolyhydroxystéarate) .

Pour le Cithrol™ DPHS, la solubilisation a été obtenue par l'utilisation d'ultrasons (Vial tweeter, 3x45s) ou en chauffant l'huile iodée.

### Vérification du sens de l'émulsion :

Une fois l'émulsion réalisée, le sens de celle-ci a été vérifié par un simple test visuel. Deux flacons ont été préparés : l'un avec du sérum physiologique et l'autre avec de l'huile iodée (Lipiodol®).

Une goutte d'émulsion fraichement préparée a été ajoutée dans chacun des deux flacons. La goutte s'est dispersée dans le flacon de Lipiodol® et ne s'est pas dispersée dans le flacon de liquide physiologique, on avait donc bien une émulsion E/H (eau-dans-huile).

La taille des gouttelettes de phase aqueuse a été évaluée à l'aide d'un microscope optique. Les gouttelettes de doxorubicine rouges étaient bien visibles au microscope dans un fond jaune d'huile.

Les principales émulsions réalisées sont décrites dans le tableau suivant :

| **Numéro du produit** | **Nature du tensioactif utilisé** | **Proportion de tensioactif utilisée (% m/v)** | **Nature de l'agent anticancére ux utilisé** | **Nature de l'agent densifiant utilisé** | **Tailles des gouttelettes de phase aqueuse** | **Stabilité visuelle observée*** |
|---|---|---|---|---|---|---|
| **E1 (Compar ative)** | PGPR | 1% | Doxorubicine | Aucun | 5 - 40 µm | Déphasage < 5% à 24h |
| **E2 (Compar ative)** | PGPR | 1% | Doxorubicine | Iobitridol** | 5 - 20 µm | Aucun déphasage à 24 h |
| **E3** | PGPR | 1% | Doxorubicine | Gadobutrol (Gadovist®) | 5 - 10µm | Aucun déphasage à 24 h |
| **E4** | PGPR | 1% | Doxorubicine | Composé X (voir formule ci-dessus) | 5 - 10µm | Aucun déphasage à 24 h |
| **E5** | PGPR | 1% | Doxorubicine | 30% en volume de Gadoteridol (ProHance ®) et 70% en volume de Iobitridol ***** | 5 - 10µm | Aucun déphasage à 24 h |
| **E6 (Compar ative)** | PGPR | 1% | Doxorubicine | Iobitridol*** | 5 - 20 µm | Aucun déphasage à 24h |
| **E7 (Compar ative)** | PGPR | 1% | Doxorubicine | Iopamidol** ** | 5 - 10µm | Aucun déphasage à 24h |
| **E8 (Compar ative)** | PGPR | 0.5% | Doxorubicine | Iobitridol** | 5 - 20 µm | |
| **E9 (Compar tive)** | PGPR | 0,3% | Doxorubicine | Iobitridol** | 5 - 20 µm | |
| **E10 (Compar ative)** | PGPR | 1% | Mitomycine C | Iobitridol** | 5 - 10 µm | Aucun déphasage à 24 h |
| **E11 (Compar ative)** | PGPR | 1% | Epirubicine | Iobitridol** | 5 - 20 µm | |
| **E12 (Compar ative)** | PGPR | 0,7% | Idarubicine | Iobitridol** | 2 - 10 µm | |
| **E13 (Compar ative)** | Cithrol™ DPHS | 1% | Doxorubicine | Aucun | 5 - 20 µm | Déphasage < 5% à 24h |
| **E14 (Compar ative)** | Cithrol™ DPHS | 1% | Doxorubicine | Iobitridol** | 5 - 20 µm | Aucun déphasage à 24h |
| **E15 (Compar ative)** | Cithrol™ DPHS | 1% | Doxorubicine | Iobitridol*** | 5 - 20 µm | |

| | | | | | | |
|---|---|---|---|---|---|---|
| * à température ambiante (20°C) ** Xenetix® 250 *** Xenetix® 300 **** Iopamiron® 250 ***** Xenetix® 350 | | | | | | |

Ces différentes émulsions préparées à l'aide d'un tensioactif de formule (I), de différents agents anticancéreux et d'agents densifiants de nature différente ont toutes démontré une stabilité conforme aux attentes.

La substitution des produits de contraste iodés tels que iobitridol et Iopamidol, utilisés comme agents densifiants, par des complexes de chélate macrocyclique non-ionique avec un ion de métal paramagnétique, n'influe pas sur la stabilité des émulsions obtenues. Grace à cette substitution, le suivi de l'efficacité des émulsions conformes à l'invention ainsi obtenues est possible par des techniques d'imagerie par résonnance magnétique comme l'IRM.

### 2. Comparaison avec des émulsions non conformes à l'invention

Des émulsions selon le même protocole que celui précisé au paragraphe 1.1 ou un protocole un peu différent (les différences par rapport au protocole du paragraphe 1.1 sont indiquées dans le tableau ci-dessous : les volumes respectifs des phases aqueuse et lipidique sont calculés à partir de leur ratio), ont été préparées en utilisant soit une concentration en PGPR non conforme à l'invention, soit des tensioactifs à HLB basse ou haute comme des tensioactifs de la famille des Span® (esters d'acides gras et de sorbitane), ou des tensioactifs de la famille des HCO (Huile de ricin hydrogénée et ethoxylée) comme l'HCO-10 (HLB = 6.5) ou l'HCO-60 (HLB = 14) fournis par la société Nikko Chemicals, des tensioactifs à HLB haute de la famille des Cremophor® (glycerol polyethylene glycol ricinoleate), de la famille des Tween® (polyoxyéthylène d'esters d'acides gras et de sorbitane) ou de la famille des Pluronics® (block copolymères à base d'oxydes d'éthylène et d'oxyde de propylène vendus par BASF) et le tensioactif CITHROL® PG32IS de HLB basse (HLB = 6,7).

D'autres agents densifiants que les complexes de chélate macrocyclique non-ionique avec un ion de métal paramagnétique ont été testés tels qu'un complexe de chélate ionique avec un ion de gadolinium (Gd-DTPA, vendu sous le nom Magnevist®) ou le PVP (polyvinylpyrrolidone), le glycérol, ou encore le dextran T40 (Sigma) mais les quantités maximales pouvant être utilisées ne permettent pas d'approcher la densité d'une huile iodée comme le Lipiodol®.

Les principales émulsions réalisées sont décrites dans les tableaux suivant :

### Emulsions préparées avec un tensioactif conforme à l'invention mais en utilisant une concentration de tensioactif non conforme et/ou un agent densifiant non conforme :

| **Numéro du produit** | **Nature du tensioactif utilisé** | **Proportion de tensioactif utilisée** | **Nature de l'agent anticancéreux utilisé** | **Ratio phase aqueuse / phase lipidique** | **Tailles des gouttelettes** | **Observations** |
|---|---|---|---|---|---|---|
| E16 | PGPR | 0,2% en masse par rapport au volume total de l'émulsion | Doxorubicine 50 mg dans 2,5 mL de Xenetix® 250 | 1/3 | Hétérogénéité : petites gouttes et des plus grosses de 20 - 50 µm | - Emulsion E/H |
| | | | | | | - Déphasage |
| E17 | PGPR | 0,2% en masse par rapport au volume total de l'émulsion | Doxorubicine 50 mg dans 2,5 mL de sérum physiologique | 1/3 | Hétérogénéité : petites gouttes et des plus grosses de 20 - 50 µm | - Emulsion E/H |
| | | | | | | - Déphasage |
| E18 | PGPR | 5% en masse par rapport au volume total de l'émulsion | Epirubicine 50 mg dans 5 mL d'eau additionnée de 290 mg de glucose (5,8% m/v) | 1/1 | Hétérogénéité : gouttes allant de 5 à 50 µm | - Emulsion H/E |
| | | | | | | - déphasage total en 2 heures |
| E19 | PGPR | 1% en masse par rapport au volume total de l'émulsion | Doxorubicine 50 mg dans 2,5 mL de Magnevist® | 1/3 | Hétérogénéité | - Emulsion E/H |
| | | | | | | - déphasage |

### Emulsions préparées avec un tensioactif et/ou un agent densifiant non conformes à l'invention et/ou dans des ratios phase aqueuse / phase lipidique non conformes :

| **Numéro du produit** | **Nature du tensioactif utilisé** | **Proportion de tensioactif utilisée** en masse par rapport au volume total de l'émulsion | **Nature de l'agent anticancére ux utilisé** | **Ratio phase aqueuse / phase lipidique** | **Tailles des gouttelettes** | **Observations** |
|---|---|---|---|---|---|---|
| E20 | Span® 80 | 1% (également testées : 0,5 et 0.8%) | 2 mL d'une solution de doxorubicine 50 mg dans 10 mL de Xenetix® 250 | 1/4 ou 1/3 | Agrégats de gouttelettes de 50 - 100 µm | - Emulsion E/H |
| | | | | | | - Déphasage quelque soit le ratio phase aqueuse/phas e lipidique et la proportion de tensioactif utilisée. |
| E21* | Span® 80 | 1% | 2 mL d'une solution de doxorubicine 50 mg dans 10 mL de sérum physiologiqu e additionné de dextan T40 à 2,5 g/50 mL | 1/4 | Hétérogénéité petites gouttes et des plus grosses de 20 - 50 µm | - Emulsion E/H |
| | | | | | | - Déphasage |
| E22* | Cremophor® EL | 0,5% | 2 mL d'une solution de doxorubicine 50 mg dans 10 mL de sérum physiologiqu e additionné de dextan T40 à 3 g/50 mL | 1/4 | 2 - 5 µm | - Emulsion E/H |
| | | | | | | - Déphasage |
| E23 | Tween® 80 | 0,1% | Doxorubicine (50 mg dans 5 mL de Xenetix® 250) | 1/1 | 10 µm | - Emulsion H/E |
| | | | | | | - Léger déphasage à 24h |
| E24* | Tween® 80 | 0,1% ou 0,01% | Doxorubicine (50 mg dans 5 mL de sérum physiologiqu e additionné de Tween® 80 et de 1% de PVP) | 1/1 | 10 - 100 µm | Emulsion H/E |
| E25* | Tween® 80 | 0,01% | Doxorubicine HCI dans 5 mL de glycérol à 2,5% | 1/1 | 100 - 300µm | - Emulsion H/E |
| | | | | | | - déphasage immédiat |
| E26' | Mélange de Tween® 80 et de Span® 80 | 0,5% | Doxorubicine (50 mg dans 2,5 mL de Xenetix® 250) | 1/3 | Hétérogénéité | Système instable non émulsionné |
| | | 0,5% | | | | |
| E27 | CITHROL® PG32IS | 1% | Doxorubicine 50 mg dans 2,5 mL de Xenetix® 250) | 1/3 | Non mesurable | - Emulsion E/H |
| | | | | | | - Déphasage instantané et violent |
| E28' | Pluronic® L 101 | 5% | Doxorubicine 50 mg dans 2,5 mL d'eau | 1/4 | 10 - 100 µm | - Emulsion H/E |
| | | | | | | - Déphasage partiel avant 18 heures |
| E29 | HCO-10 | 1% | Doxorubicine 50 mg dans 2,5 mL de Xenetix® 250) | 1/3 | | - Emulsion E/H |
| | | | | | | - Déphasage en 10 heures |
| E30 | HCO-60 | 1% | Doxorubicine 50 mg dans 2,5 mL de Xenetix® 250) | 1/3 | | - Emulsion E/H |
| | | | | | | - Déphasage rapide |
| E31 | HCO-60 | 1% | Doxorubicine 50 mg dans 5 mL de sérum physiologiqu e | 1/1 | | - Emulsion H/E |

| | | | | | | |
|---|---|---|---|---|---|---|
| * émulsion préparée avec un agent densifiant non conforme à l'invention : PVP (polyvinylpyrrolidone), Dextran T40, acide ioxaglique (Hexabrix®) ou glycérol | | | | | | |

### Emulsions préparées sans tensioactif et/ou sans agent densifiant :

| **Numéro du produit** | **Nature de l'agent anticancéreux utilisé** | **Ratio phase aqueuse / phase lipidique** | **Tailles des gouttelettes** | **Observations** |
|---|---|---|---|---|
| E32* | Doxorubicine (50 mg dans 5 mL de sérum physiologique) | 1/1 | 10 µm | Emulsion H/E |
| E33 | Doxorubicine (50 mg dans 5 mL de Xenetix® 250) | 1/1 | 10 µm | Emulsion H/E |
| E34* | Doxorubicine (50 mg dans 2,5 mL de Xenetix® 250) | 1/3 ou 1/2 | Non mesurable (supérieure à 200 µm) | - Emulsion E/H |
| | | | | - Déphasage immédiat |
| E35* | Doxorubicine (50 mg dans 3 mL de sérum physiologique) | 1/4 | 10 µm | - Emulsion H/E |
| | | | | - Emulsion très visqueuse et épaisse : non utilisable dans un cadre clinique. |
| E36 | Epirubicine (50 mg dans 5 mL de Iopamiron® 250) | 1/1 | 10 µm | Emulsion H/E |
| E37* | Epirubicine (50 mg dans 5 mL de sérum physiologique) | 1/1 | 10 - 20 µm | Emulsion H/E |

| | | | | |
|---|---|---|---|---|
| émulsion préparée sans agent densifiant | | | | |

Le Span® 80 (Croda) est du monooléate de sorbitane. Le Tween® 80 (Croda), aussi appelé polysorbate 80, est du PEG-20 monoléate de sorbitane. Le Cremophor® EL (BASF) a comme nom chimique : polyoxyl 35 Castor Oil. Le CITHROL® PG32IS est du polyglyceryl-3-diisostéarate. Il n'est donc pas ramifié comme les tensioactifs de formule (I). L'HCO-10 (HLB = 6.5) porte le nom INCI : PEG-10 HYDROGENATED CASTOR OIL tandis que l'HCO-60 (HLB = 14) porte le nom INCI: PEG-60 HYDROGENATED CASTOR OIL.

D'autres Span® que le Span® 80 (HLB = 4,3) ont été testés : Span® 20 (HLB = 8,6), Span® 65 (HLB = 2,1), Span® 83 (HLB = 3,7), Span® 85 (HLB = 1,8). Les émulsions préparées avec ces tensioactifs ont toutes déphasé aussitôt après leur préparation. Les essais réalisés avec des composés Pluronic® (BASF) n'ont pas non plus été concluants puisque l'on n'a pas pu réaliser une émulsion avec ces composés.

Ainsi, l'ensemble des émulsions comparatives obtenues ont présenté soit des stabilités insuffisantes, soit un sens non conforme à l'invention, soit n'étaient pas visibles (ou étaient insuffisamment visibles) par des techniques d'imagerie par résonance magnétique comme l'IRM.

## Revendications

1. Composition sous forme d'émulsion eau-dans-huile comprenant :
- de 20 à 40% (v/v) de phase aqueuse, sous forme de gouttelettes, comprenant un agent anticancéreux et un agent densifiant choisi parmi les complexes de chélate macrocyclique non-ionique avec un métal paramagnétique,
- de 60 à 80% (v/v) de phase lipidique comprenant une huile iodée et au moins un tensioactif de formule (I) dans une proportion, en masse de tensioactif par rapport au volume total de la composition, de 0,3 à 5%, la formule (I) dudit tensioactif étant la suivante: 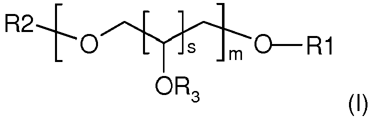 dans laquelle :
- s vaut 0 ou 1,
- m représente un nombre entier de 2 à 30,
- R₁ représente un groupement de formule (II) 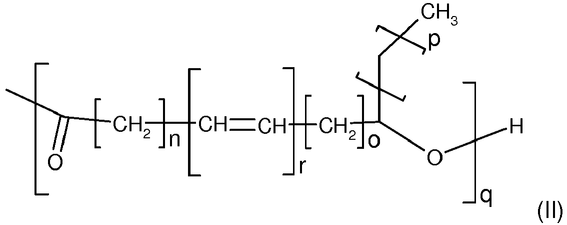 dans laquelle n représente un nombre entier de 4 à 10, o représente un nombre entier de 1 à 4, p représente un nombre entier de 3 à 7, q représente un nombre entier de 2 à 10 et r vaut 0 ou 1,
- R₂ représente un atome d'hydrogène ou est identique à R₁, et
- chaque R₃ représente indépendamment un atome d'hydrogène ou est identique à R₁.

2. Composition selon la revendication 1, dans laquelle chaque R₃ représente un atome d'hydrogène.

3. Composition selon la revendication 1 ou 2, **caractérisée en ce qu'**elle présente, à 20°C et à 1 bar, et dans les 24 heures suivant sa préparation, un déphasage visuel de moins de 5% en volume par rapport à la totalité de la composition, ou **caractérisée en ce que** la taille moyenne des gouttelettes varie de moins de 10% 24 heures après sa préparation, où la taille moyenne est mesurée au microscope optique.

4. Composition selon l'une quelconque des revendications 1 à 3, **caractérisée en ce que** l'agent anticancéreux est choisi parmi les anthracyclines, les complexes de platine, la mitoxantrone, la nemorubicine, la mitomycine C, la bleomycine, l'actinomycine D, l'irinotecan, le 5-Fluoro-Uracile, le sorafénib, le sunitinib, le regorafénib, le brivanib, l'orantinib, le linsitinib, l'erlotinib, le cabozantinib, le foretinib, le tivantinib, la fotémustine, la tauromustine (TCNU), la carmustine, la cytosine C, le cyclophosphonamide, la cytosine arabinoside, le paclitaxel, le docétaxel, le methotrexate, l'everolimus, le PEG-arginine deiminase, la combinaison tegafur/gimeracil/oteracil, le muparfostat, le pérétinoine, la gemcitabine, le bévacizumab, et le ramucirumab, la floxuridine, le GM-CSF, la molgramostim, la sargramostim, l'OK-432, l'interleukine-2, l'interleukine-4 et le TNFalpha, les anticorps anti-CEA (CarcinoEmbryonic Antigen) marqués à l'¹²⁵I, les microsphères chargées en l'un de ces composés, les radio-éléments et les complexes desdits radioéléments avec des chélates macrocycliques, les particules magnétiques à base d'un composé de fer et/ou d'un chélate de gadolinium, les microsphères radioactives, les séquences d'acide nucléique choisies parmi des séquences d'acide désoxyribonucléique et d'acide ribonucléique et un mélange de ceux-ci.

5. Composition selon la revendication précédente, **caractérisée en ce que** les anthracyclines sont choisies parmi la doxorubicine, l'épirubicine, la némorubicine et l'idarubicine.

6. Composition selon l'une quelconque des revendications 1 à 5, **caractérisée en ce que** les complexes de chélate macrocyclique non-ionique avec un métal paramagnétique sont choisis parmi le Gd-HP-DO3A, le Gd-BT-DO3A, un complexe de formule (XI) : 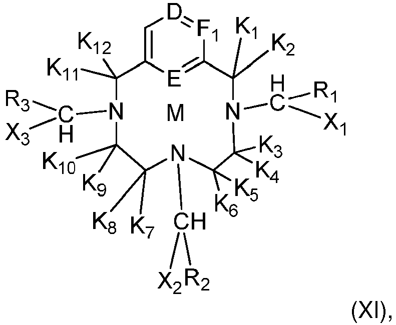 dans laquelle R₁, R₂ et R₃ représentent -COOH,
X₁, X₂ et X₃ représentent indépendamment l'un de l'autre L-Y dans laquelle L représente un groupe alkylène en C₁-C₃, de préférence (CH₂)ₙ avec n = 1 à 3, Y représente -CONH₂, -CO-NR₇R₈ ou -NR₇-CO-R₈, dans lesquelles R₇ représente H ou un groupe alkyle en C₁-C₆ ou un groupe hydroxyalkyle en C₁-C₈, notamment en C₂-C₆, par exemple en C₂-C₄, avantageusement -CH₂-CH₂OH, -CHOH-CH₂OH, -CH-(CH₂OH)₂, -(CH₂)ₘ-(CHOH)ₚ-CH₂OH avec m = 1 à 3 et p = 1 à 4 ou -C-(CH₂OH)₃ et R₈ représente un groupe alkyle en C₁-C₆ ou hydroxyalkyle en C₁-C₈, notamment en C₂-C₆, par exemple en C₂-C₄, avantageusement -CH₂-CH₂OH, -CHOH-CH₂OH, -CH-(CH₂OH)₂, - (CH₂)ₘ-(CHOH)ₚ-CH₂OH avec m = 1 à 3 et p = 1 à 4 ou -C-(CH₂OH)₃, à la condition qu'au moins R₇ ou R₈ représente un groupe hydroxyalkyle en C₁-C₈ ;
D représente CH ou N ;
E représente CH ou N ;
F₁ représente CH ou N ;
K₁ à K₁₂ représentent chacun indépendamment H, -(CH₂)ⱼ-CH₃ ou -(CH₂)ᵢ-OH dans lesquels j = 0 à 3 et i = 1 à 3, avantageusement H, ou K₃ ou K₄ avec K₅ ou K₆, et/ou K₇ ou K₈ avec K₉ ou K₁₀ forment avec les atomes de carbone auxquels ils sont liés un cycle ayant 3 à 6 atomes de carbone ; et
M représente un ion d'un métal paramagnétique ;
ou un énantiomère, ou un diastéréoisomère (préférentiellement choisi parmi les diastéréoisomères RRS, RSR, RSS) de ceux-ci ou leurs mélanges,
et un mélange de ceux-ci.

7. Composition selon l'une quelconque des revendications 1 à 6, **caractérisée en ce que** la phase lipidique comprend en outre une huile non iodée choisie parmi l'huile de lin, l'huile de soja, l'huile de palme, l'huile de coco, l'huile de ricin, l'huile de maïs, l'huile de coton, l'huile d'arachide, l'huile de sésame, l'huile de tournesol, l'huile de carthame, l'huile d'amande, l'huile d'olive, l'huile de pavot et une huile comprenant ou consistant en un mélange de triglycérides d'acide gras de formule : 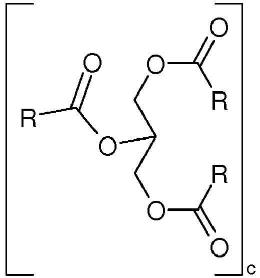 où R est une chaîne aliphatique comprenant de 3 à 35 atomes de carbone, sous réserve que plus de 95% desdits acides gras soient en C8 et/ou en C10.

8. Composition selon l'une quelconque des revendications précédentes, **caractérisée en ce que** le tensioactif a une HLB de 1 à 8.

9. Composition selon l'une quelconque des revendications précédentes, **caractérisée en ce que** le tensioactif est choisi parmi le polyricinoléate de polyglycérol et le PEG-30-dipolyhydroxystéarate.

10. Composition selon l'une quelconque des revendications précédentes, **caractérisée en ce que** l'huile iodée comprend des esters éthyliques d'acides gras iodés d'huile d'œillette ou d'huile d'olive.

11. Composition selon l'une quelconque des revendications 1 à 10, **caractérisée en ce que** la taille des gouttelettes de la phase aqueuse est de 1 à 200 µm.

12. Composition selon l'une quelconque des revendications précédentes, **caractérisée en ce qu'**elle présente une viscosité à 20°C comprise de 100 et 200 mPa.s et/ou une viscosité à 37°C comprise de 40 et 80 mPa.s.

13. Composition selon l'une quelconque des revendications 1 à 12, pour son utilisation dans le traitement du cancer ou de ses métastases.

14. Procédé de préparation d'une composition selon l'une quelconque des revendications 1 à 13, comprenant les étapes suivantes :
a) Mélange du tensioactif tel que défini à la revendication 1 ou 2 dans l'huile iodée, et
b) Mélange de la solution obtenue à l'étape a) avec une solution aqueuse comprenant un agent anticancéreux et un agent densifiant choisi parmi les complexes de chélate macrocyclique non-ionique avec un métal paramagnétique et un mélange de ceux-ci.

15. Composition selon l'une quelconque des revendications 1, 2, 3, 8 à 10 et 12 pour son utilisation comme vecteur d'un agent anticancéreux.

## Patentansprüche

1. Zusammensetzung in Form einer Wasser-in-Öl-Emulsion, umfassend:
- 20 bis 40 % (v/v) wässrige Phase in Form von Tröpfchen, umfassend ein Antikrebsmittel und ein Verdichtungsmittel, ausgewählt aus nichtionischen makrozyklischen Chelatkomplexen mit einem paramagnetischen Metall,
- 60 bis 80 % (v/v) Lipidphase, umfassend ein iodiertes Öl und mindestens ein Tensid der Formel (I) in einem Gewichtsanteil an Tensid, bezogen auf das Gesamtvolumen der Zusammensetzung, von 0,3 bis 5 %, wobei das Tensid die folgende Formel (I) hat: 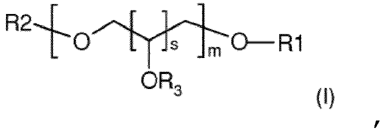 worin:
- s den Wert 0 oder 1 hat,
- m eine ganze Zahl von 2 bis 30 darstellt,
- R₁ eine Gruppe der folgenden Formel (II) darstellt 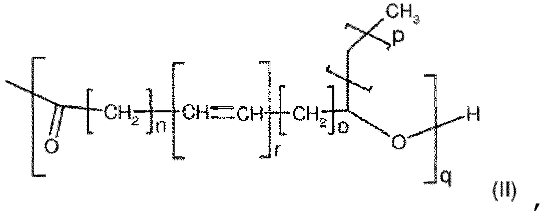 worin n eine ganze Zahl von 4 bis 10 darstellt, o eine ganze Zahl von 1 bis 4 darstellt, p eine ganze Zahl von 3 bis 7 darstellt, q eine ganze Zahl von 2 bis 10 darstellt und r den Wert 0 oder 1 hat,
- R₂ ein Wasserstoffatom darstellt oder identisch mit R₁ ist und
- jedes R₃ unabhängig ein Wasserstoffatom darstellt oder identisch mit R₁ ist.

2. Zusammensetzung nach Anspruch 1, wobei jedes R₃ ein Wasserstoffatom darstellt.

3. Zusammensetzung nach Anspruch 1 oder 2, **dadurch gekennzeichnet, dass** sie bei 20 °C und 1 bar und innerhalb der 24 Stunden nach ihrer Herstellung eine sichtbare Phasentrennung von weniger als 5 Vol.-%, bezogen auf die Gesamtheit der Zusammensetzung, aufweist, oder dadurch, dass die mittlere Größe der Tröpfchen 24 Stunden nach ihrer Herstellung um weniger als 10 % abweicht, wobei die mittlere Größe mit einem optischen Mikroskop gemessen wird.

4. Zusammensetzung nach einem der Ansprüche 1 bis 3, **dadurch gekennzeichnet, dass** das Antikrebsmittel ausgewählt ist aus Anthracyclinen, Platinkomplexen, Mitoxantron, Nemorubicin, Mitomycin C, Bleomycin, Actinomycin D, Irinotecan, 5-Fluoruracil, Sorafenib, Sunitinib, Regorafenib, Brivanib, Orantinib, Linsitinib, Erlotinib, Cabozantinib, Foretinib, Tivantinib, Fotemustin, Tauromustin (TCNU), Carmustin, Cytosin C, Cyclophosphonamid, Cytosin-Arabinosid, Paclitaxel, Docetaxel, Methotrexat, Everolimus, PEG-Arginin-Deiminase, der Kombination Tegafur/Gimeracil/Oteracil, Muparfostat, Peretinoin, Gemcitabin, Bevacizumab und Ramucirumab, Floxuridin, GM-CSF, Molgramostim, Sargramostim, OK-432, Interleukin-2, Interleukin-4 und TNFalpha, ¹²⁵I-markierten Anti-CEA(carcinoembryonales Antigen)-Antikörpern, Mikrosphären, die mit einer dieser Verbindungen beladen sind, Radioelementen und Komplexen der Radioelemente mit makrozyklischen Chelaten, magnetischen Partikeln auf der Basis einer Eisenverbindung und/oder eines Gadoliniumchelats, radioaktiven Mikrosphären, Nukleinsäuresequenzen, ausgewählt aus Desoxyribonukleinsäuresequenzen und Ribonukleinsäuresequenzen, und einem Gemisch von diesen.

5. Zusammensetzung nach dem vorhergehenden Anspruch, **dadurch gekennzeichnet, dass** die Anthracycline aus Doxorubicin, Epirubicin, Nemorubicin und Idarubicin ausgewählt sind.

6. Zusammensetzung nach einem der Ansprüche 1 bis 5, **dadurch gekennzeichnet, dass** die nichtionischen makrozyklischen Chelatkomplexe mit einem paramagnetischen Metall ausgewählt sind aus Gd-HP-DO3A, Gd-BT-LO3A, einem Komplex der Formel (XI): 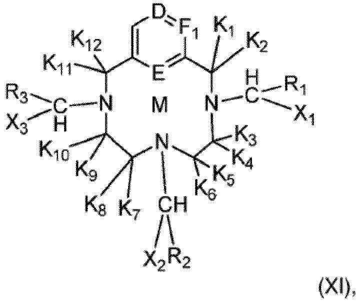
worin R₁, R₂ und R₃ -COOH darstellen,
X₁, X₂ und X₃ unabhängig voneinander L-Y darstellen, wobei L eine C₁-C₃-Alkylengruppe, vorzugsweise (CH₂)ₙ mit n = 1 bis 3, darstellt, Y -CONH₂, -CO-NR-₇R₈ oder -NR₇-CO-R₈ darstellt, worin R₇ H oder eine C₁-C₆-Alkyl- oder eine C₁-C₈-, insbesondere C₂-C₆-, zum Beispiel C₂-C₄-Hydroxyalkylgruppe, vorteilhafterweise -CH₂-CH₂OH, -CHOH-CH₂OH, -CH-(CH₂OH)₂, - (CH₂)ₘ- (CHOH)ₚ-CH₂OH mit m = 1 bis 3 und p = 1 bis 4 oder -C-(CH₂OH)₃ darstellt und R₈ eine C₁-C₆-Alkyl- oder C₁-C₈-, insbesondere C₂-C₆-, zum Beispiel C₂-C₄-Hydroxyalkylgruppe, vorteilhafterweise -CH₂-CH₂OH, -CHOH-CH₂OH, -CH- (CH₂OH)₂, - (CH₂)ₘ- (CHOH) ₚ-CH₂OH mit m = 1 bis 3 und p = 1 bis 4 oder -C-(CH₂OH)₃ darstellt, mit der Maßgabe, dass mindestens R₇ oder R₈ eine C₁-C₈-Hydroxyalkylgruppe darstellt,
D CH oder N darstellt,
E CH oder N darstellt,
F₁ CH oder N darstellt,
K₁ bis K₁₂ jeweils unabhängig H, -(CH₂) ⱼ-CH₃ oder -(CH₂)i-OH, worin j = 0 bis 3 und i = 1 bis 3, vorteilhafterweise H darstellen oder K₃ oder K₄ mit K₅ oder K₆ und/oder K₇ oder K₈ mit K₉ oder K₁₀ mit den Kohlenstoffatomen, an die sie gebunden sind, einen Ring mit 3 bis 6 Kohlenstoffatomen bilden und
M ein Ion eines paramagnetischen Metalls darstellt,
oder einem Enantiomer oder einem Diastereomer (vorzugsweise ausgewählt aus RRS-, RSR, RSS-Diastereomeren) von diesen oder deren Gemischen,
und einem Gemisch davon.

7. Zusammensetzung nach einem der Ansprüche 1 bis 6, **dadurch gekennzeichnet, dass** die Lipidphase ferner ein nicht iodiertes Öl umfasst, ausgewählt aus Leinöl, Sojaöl, Palmöl, Kokosöl, Rizinusöl, Maisöl, Baumwollsamenöl, Erdnussöl, Sesamöl, Sonnenblumenöl, Saffloröl, Mandelöl, Olivenöl, Mohnöl und einem Öl, umfassend ein oder bestehend aus einem Gemisch von Fettsäuretriglyceriden der Formel: 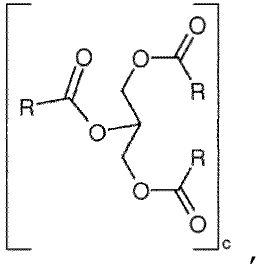 worin R eine aliphatische Kette mit 3 bis 35 Kohlenstoffatome ist, mit der Maßgabe, dass mehr als 95 % dieser Fettsäuren C₈- und/oder C₁₀-Fettsäuren sind.

8. Zusammensetzung nach einem der vorhergehenden Ansprüche, **dadurch gekennzeichnet, dass** das Tensid einen HLB-Wert von 1 bis 8 aufweist.

9. Zusammensetzung nach einem der vorhergehenden Ansprüche, **dadurch gekennzeichnet, dass** das Tensid aus Polyglycerin-Polyricinoleat und PEG-30-Dipolyhydroxystearat ausgewählt ist.

10. Zusammensetzung nach einem der vorhergehenden Ansprüche, **dadurch gekennzeichnet, dass** das iodierte Öl iodierte Mohnsamenöl- oder Olivenöl-Fettsäureethylester umfasst.

11. Zusammensetzung nach einem der Ansprüche 1 bis 10, **dadurch gekennzeichnet, dass** die Größe der Tröpfchen der wässrigen Phase 1 bis 200 µm beträgt.

12. Zusammensetzung nach einem der vorhergehenden Ansprüche, **dadurch gekennzeichnet, dass** sie eine Viskosität bei 20 °C von 100 und 200 mPa.s und/oder eine Viskosität bei 37 °C von 40 und 80 mPa.s aufweist.

13. Zusammensetzung nach einem der Ansprüche 1 bis 12 zur Verwendung bei der Behandlung von Krebs oder dessen Metastasen.

14. Verfahren zur Herstellung einer Zusammensetzung nach einem der Ansprüche 1 bis 13, das die folgenden Schritte umfasst:
a) Mischen des Tensids nach Anspruch 1 oder 2 in das iodierte Öl und
b) Mischen der in Schritt a) erhaltenen Lösung mit einer wässrigen Lösung, die ein Antikrebsmittel und ein Verdichtungsmittel, ausgewählt aus nichtionischen makrozyklischen Chelatkomplexen mit einem paramagnetischen Metall und einem Gemisch von diesen, umfasst.

15. Zusammensetzung nach einem der Ansprüche 1, 2, 3, 8 bis 10 und 12 zur Verwendung als Vehikel für ein Antikrebsmittel.

## Claims

1. Composition in the form of a water-in-oil emulsion comprising:
- from 20% to 40% (v/v) of aqueous phase, in the form of droplets, comprising an anti-cancer agent and a densifying agent chosen from complexes of a nonionic macrocyclic chelate with a paramagnetic metal,
- from 60% to 80% (v/v) of lipid phase comprising an iodized oil and at least one surfactant of formula (I) in a proportion, by weight of surfactant relative to the total volume of the composition, of 0.3% to 5%, formula (I) of said surfactant being the following: 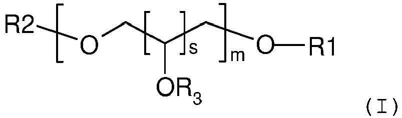 in which:
- s is 0 or 1,
- m represents an integer from 2 to 30,
- R₁ represents a group of formula (II) 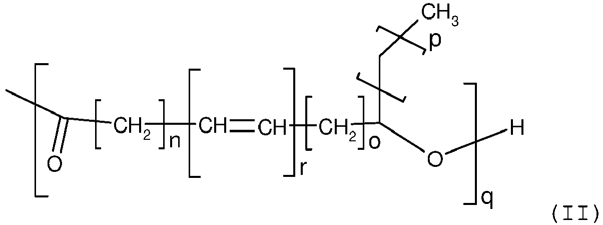 in which n represents an integer from 4 to 10, o represents an integer from 1 to 4, p represents an integer from 3 to 7, q represents an integer from 2 to 10 and r is 0 or 1,
- R₂ represents a hydrogen atom or is identical to R₁, and
each R₃ independently represents a hydrogen atom or is identical to R₁.

2. Composition according to Claim 1, in which each R₃ represents a hydrogen atom.

3. Composition according to Claim 1 or 2, **characterized in that** it exhibits, at 20°C and at 1 bar, and within 24 hours following the preparation thereof, a visual phase separation of less than 5% by volume relative to the entire composition, or **characterized in that** the average droplet size varies by less than 10% 24 hours after the preparation of said composition, wherein the average size is measured under an optical microscope.

4. Composition according to any one of Claims 1 to 3, **characterized in that** the anti-cancer agent is chosen from anthracyclines, platinum complexes, mitoxantrone, nemorubicin, mitomycin C, bleomycin, actinomycin D, irinotecan, 5-fluorouracil, sora-fenib, sunitinib, regorafenib, brivanib, orantinib, linsitinib, erlotinib, cabozantinib, foretinib, tivantinib, fotemustine, tauromustine (TCNU), carmustine, cytosine C, cyclophosphonamide, cytosine arabinoside, paclitaxel, docetaxel, methotrexate, everolimus, PEG-arginine deiminase, the tegafur/gimeracil/oteracil combination, muparfostat, peretinoin, gemcitabine, bevacizumab and ramucirumab, floxuridine, GM-CSF, molgramostim, sargramostim, OK-432, interleukin-2, interleukin-4 and TNFalpha, ¹²⁵I-labelled anti-CEA (carcino-embryonic antigen) antibodies, microspheres loaded with one of these compounds, radioelements and complexes of said radioelements with macrocyclic chelates, magnetic particles based on an iron compound and/or on a gadolinium chelate, radioactive microspheres, nucleic acid sequences chosen from deoxyribonucleic acid and ribonucleic acid sequences and a mixture thereof.

5. Composition according to the preceding claim, **characterized in that** the anthracyclines are chosen from doxorubicin, epirubicin, nemorubicin and idarubicin.

6. Composition according to any one of Claims 1 to 5, **characterized in that** the complexes of a non-ionic macrocyclic chelate with a paramagnetic metal are chosen from Gd-HP-DO3A, Gd-BT-DO3A, a complex of formula (XI): 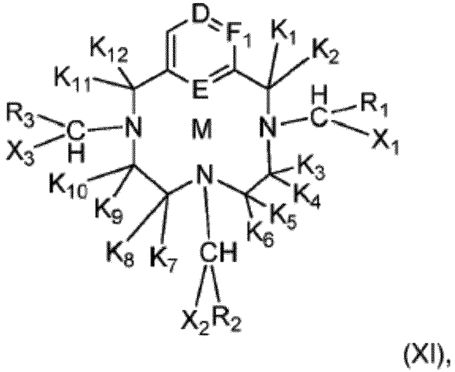 in which R₁, R₂ and R₃ represent -COOH,
X₁, X₂ and X₃ represent, independently of one another, L-Y in which L represents a C₁-C₃ alkylene group, preferably (CH₂)ₙ with n = 1 to 3, Y represents -CONH₂, -CO-NR-₇R₈ or -NR-₇-CO-R₈, in which R₇ represents H or a C₁-C₆ alkyl group or a C₁-C₈, in particular C₂-C₆, for example C₂-C₄, hydroxyalkyl group, advantageously -CH₂-CH₂OH, -CHOH-CH₂OH, -CH-(CH₂OH)₂, - (CH₂)ₘ-(CHOH)ₚ-CH₂OH with m = 1 to 3 and p = 1 to 4 or -C-(CH₂OH)₃ and R₈ represents a C₁-C₆ alkyl group or a C₁-C₈, in particular C₂-C₆, for example C₂-C₄, hydroxyalkyl group, advantageously -CH₂-CH₂OH, -CHOH-CH₂OH, -CH-(CH₂OH)₂, - (CH₂)ₘ-(CHOH)ₚ-CH₂OH with m = 1 to 3 and p = 1 to 4 or -C-(CH₂OH)₃, on the condition that at least R₇ or R₈ represents a C₁-C₈ hydroxyalkyl group;
D represents CH or N;
E represents CH or N;
F₁ represents CH or N;
K₁ to K₁₂ each independently represent H, -(CH₂) ⱼ-CH₃ or -(CH₂)i-OH in which j = 0 to 3 and i = 1 to 3, advantageously H, or K₃ or K₄ with K₅ or K₆, and/or K₇ or K₈ with K₉ or K₁₀ form, with the carbon atoms to which they are attached, a ring containing 3 to 6 carbon atoms; and
M represents an ion of a paramagnetic metal;
or an enantiomer, or a diastereoisomer (preferentially chosen from the diastereoisomers RRS, RSR, RSS) thereof, or mixtures thereof,
and a mixture thereof.

7. Composition according to any one of Claims 1 to 6, **characterized in that** the lipid phase also comprises a non-iodized oil chosen from linseed oil, soybean oil, palm oil, coconut oil, castor oil, maize oil, cottonseed oil, peanut oil, sesame oil, sunflower oil, safflower oil, almond oil, olive oil, poppy oil and an oil comprising or consisting of a mixture of fatty acid triglycerides of formula: 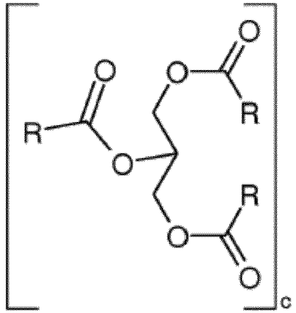 where R is an aliphatic chain comprising from 3 to 35 carbon atoms, with the proviso that more than 95% of said fatty acids are Cs and/or C₁₀ fatty acids.

8. Composition according to any one of the preceding claims, **characterized in that** the surfactant has an HLB of 1 to 8.

9. Composition according to any one of the preceding claims, **characterized in that** the surfactant is chosen from polyglycerol polyricinoleate and PEG-30 dipolyhydroxystearate.

10. Composition according to any one of the preceding claims, **characterized in that** the iodized oil comprises ethyl esters of iodized fatty acids of poppyseed oil or of olive oil.

11. Composition according to any one of Claims 1 to 10, **characterized in that** the size of the aqueous phase droplets is from 1 to 200 pm.

12. Composition according to any one of the preceding claims, **characterized in that** it has a viscosity at 20°C of from 100 and 200 mPa.s and/or a viscosity at 37°C of from 40 and 80 mPa.s.

13. Composition according to any one of Claims 1 to 12, for use thereof in the treatment of cancer or metastases thereof.

14. Method for preparing a composition according to any one of Claims 1 to 13, comprising the following steps:
a) mixing the surfactant as defined in Claim 1 or 2 in the iodized oil, and
b) mixing the solution obtained in step a) with an aqueous solution comprising an anti-cancer agent and a densifying agent chosen from complexes of a non-ionic macrocyclic chelate with a paramagnetic metal and a mixture thereof.

15. Composition according to any one of Claims 1, 2, 3, 8 to 10 and 12 for use thereof as an anti-cancer agent vector.
